# EUROPÄISCHE PATENTSCHRIFT

(11) **EP 3 284 514 B1**
(45) Veröffentlichungstag und Bekanntmachung des Hinweises auf die Patenterteilung: **27.03.2019**
(21) Anmeldenummer: 16184556.5
(22) Anmeldetag: 17.08.2016
(51) Int. Cl.: A61N 1/375

(54) **ELEKTRISCHE DURCHFÜHRUNG, INSBESONDERE CERMETDURCHFÜHRUNG, MIT EINSTÜCKIGEM DURCHFÜHRUNGSELEMENT AUS MEHREREN BEREICHEN, INSBESONDERE ZUM VERBESSERTEN KONTAKTIEREN DER DURCHFÜHRUNG**
ELECTRICAL FEEDTHROUGH, IN PARTICULAR CERMET FEEDTHROUGH, WITH SINGLE PIECE FEEDTHROUGH ELEMENT MADE FROM MULTIPLE AREAS, IN PARTICULAR FOR IMPROVED CONTACTING OF THE FEEDTHROUGH
PASSAGE ELECTRIQUE, EN PARTICULIER PASSAGE DE CERMET COMPRENANT UN ELEMENT DE PASSAGE EN UN SEUL TENANT A PARTIR DE PLUSIEURS ZONES, EN PARTICULIER POUR AMELIORER LA MISE EN CONTACT DU PASSAGE

(43) Veröffentlichungstag der Anmeldung: 21.02.2018
(73) Patentinhaber: Heraeus Deutschland GmbH & Co. KG, 63450 Hanau (DE)
(72) Erfinder: Nikolaidis, Ilias, 63450 Hanau (DE); Hausch, Ulrich, 60318 Frankfurt (DE); Roth, Frederik, 63450 Hanau (DE); Dittmer, Robert, 63450 Hanau (DE); Gebhardt, Jacqueline, 71409 Schwaikheim (DE); Sauer, Sandra, 63762 Großostheim (DE)
(74) Vertreter: Herzog, Fiesser & Partner Patentanwälte PartG mbB

(56) Entgegenhaltungen:
- US-A1- 2012 193 117
- US-A1- 2012 193 125
- US-A1- 2015 165 219

## Beschreibung

Die vorliegende Erfindung betrifft eine Vorrichtung, beinhaltend einen Keramikkörper mit einem Tunnel und einer Tunnelfüllung, beinhaltend einen ersten Bestandteil und einen zweiten Bestandteil, wobei sich die elektrische Leitfähigkeit des ersten Bestandteils und des zweiten Bestandteils um mindestens 5·10⁴ S/m unterscheidet; ein elektrisches Gerät, insbesondere ein implantierbares elektrisches medizinisches Gerät; und eine Verwendung der vorgenannten Vorrichtung.

Im Stand der Technik sind zahlreiche implantierbare elektrische medizinische Geräte bekannt, so zum Beispiel Schrittmacher, Defibrillatoren und Elektrokardiogrammgeräte (EKG-Geräte). Zu den bekannten Schrittmachern gehören Blasenschrittmacher, Atemschrittmacher, Darmschrittmacher, Zwerchfellschrittmacher, Hirnschrittmacher und besonders Herzschrittmacher. Solche Geräte werden typischerweise in einen menschlichen oder tierischen Organismus implantiert, um eine Krankheit oder Fehlfunktion des Organismus zu therapieren, zu behandeln oder zu überwachen. Hierzu muss stets eine elektrische Verbindung zwischen dem Geräteinneren und einer Umgebung des Gerätegehäuses bestehen. So soll ein Schrittmacher im Einsatzfall über einen elektrischen Impulsgeber im Inneren des Schrittmachergehäuses einen elektrischen Spannungspuls erzeugen und diesen über eine Elektrode außerhalb des Schrittmachergehäuses an organisches Gewebe des Patienten abgeben. Ein implantiertes Messgerät wie ein EKG-Gerät oder Biomonitor nimmt über Messelektroden außerhalb des Gerätegehäuses elektrische Spannungssignale auf und leitet diese zur Verarbeitung in das Gehäuse. In jedem Fall muss eine elektrische Verbindung, oft eine Vielzahl elektrischer Verbindungen zwischen dem Inneren und der Umgebung des Gehäuses bereitgestellt werden. Dabei muss jedoch sicher gestellt sein, dass das Gehäuseinnere von der organischen Umgebung hermetisch dicht abgeschlossen ist. Hierzu werden im Stand der Technik elektrische Durchführungen eingesetzt. Eine elektrische Durchführung des Stands der Technik beinhaltet ein elektrisch leitendes Durchführungselement, meist ein Stift oder ein Draht aus Platin, welches sich vom Geräteinneren bis nach außen erstreckt. Dabei ist das Durchführungselement von dem meist aus einer Titanlegierung bestehenden Gehäuse elektrisch isoliert, meist durch einen Keramikring. Die benötigte hermetische Dichtigkeit der Durchführung wird meist dadurch erreicht, dass ein Titanflansch in eine Öffnung des Gehäuses eingeschweißt wird und der Keramikring in diesen Flansch eingeschweißt oder gelötet wird. Das Durchführungselement ist mit einem Goldlot in den Keramikring eingelötet. Um die hermetische Dichtigkeit zu erreichen, werden demnach im Stand der Technik unter Einsatz kostspieliger Materialien wie dem Goldlot zahlreiche Verbindungen zwischen verschiedenen Komponenten und Materialien erzeugt. Dies führt zu einem sehr aufwendigen und teuren Herstellungsverfahren sowie zu Nachteilen bei der Zuverlässigkeit des Geräts. Die verschiedenen Verbindungsstellen sind potentielle Bruch- und Undichtigkeitsstellen. Eine Undichtigkeit eines implantierten elektrischen medizinischen Geräts muss jedoch unbedingt vermieden werden. Wird ein solches Gerät undicht kann es dem Patienten schaden oder das Gerät fällt aus und kann seine Funktion nicht mehr erfüllen. Dies ist in Anbetracht der durch einen Schrittmacher zu therapierenden Krankheiten, wie zum Beispiel Herzleiden, besonders schwerwiegend. Selbst wenn Störungen des Geräts rechtzeitig erkannt werden bedeutet ein Austauschen des Geräts einen erheblichen chirurgischen Eingriff in den Patientenkörper.

In diesem Zusammenhang zeigen Cermetdurchführungen erhebliche Vorteile. Zu ihrer Herstellung wird ein Durchführungskanal in einem Keramikgrünling mit einer Cermetpaste gefüllt und beides zusammen gebrannt. Somit werden der Isolationskörper aus Keramik sowie das elektrisch leitende Durchführungselement in einem Stück ohne Intermaterialverbindungen hergestellt. Um eine geeignete Dichte des Cermetdurchführungselements, ein geeignetes thermisches Expansionsverhalten sowie eine gute Bindung zu dem Keramikkörper zu erzielen, ist es vorteilhaft ein Cermet mit nicht zu hohem Metallanteil, meist ein Anteil von Platin, zu verwenden. Um nun das Cermetdurchführungselement als solches nutzen zu können, muss es an beiden Enden, im Gehäuseinneren wie auch an der dem Patientenkörper zugwandten Seite, elektrisch kontaktiert werden durch Löten, Bonden oder Schweißen. Damit eine solche elektrische Verbindung gelingt, wird an dem Durchführungselement eine Oberfläche mit erhöhtem Metallanteil bzw. einer erhöhten elektrischen Leitfähigkeit benötigt. Eine solche Oberfläche wird im Stand der Technik nachträglich zusätzlich auf das Durchführungselement aufgebracht, zum Beispiel durch Drucken einer Paste und anschließendem Brennen oder Sintern, durch thermisches Spritzen, oder durch Dünnfilmbeschichten. In jedem Fall wird durch zusätzliche Verfahrensschritte auf jeder Seite des Durchführungselements mindestens eine zusätzlich Intermaterialverbindung erzeugt. Dies läuft der Grundidee einer einstückigen Cermetdurchführung aber gerade zuwider. Der Herstellungsprozess der Cermetdurchführung des Stands der Technik wird somit aufwendiger und teurer. Ferner machen die zusätzlichen Verbindungsstellen die Durchführung des Stands der Technik mechanisch weniger robust und haltbar.

Eine elektrische Durchführung, deren Cermetdurchführungselement wie oben beschrieben durch Schweißen mittels einer zusätzlich dafür aufgebrachten Metallschicht elektrisch kontaktiert wird, ist in US 2012/0193125 A1 offenbart.

Allgemein ist es eine Aufgabe der vorliegenden Erfindung, einen Nachteil, der sich aus dem Stand der Technik ergibt, zumindest teilweise zu überwinden. Eine weitere Aufgabe der Erfindung ist es, eine elektrisch kontaktierbare elektrische Durchführung, insbesondere eine Cermetdurchführung, mit einer erhöhten mechanischen Robustheit, insbesondere von elektrischen Kontaktstellen, bereitzustellen. Eine weitere Aufgabe der Erfindung ist es, eine elektrisch kontaktierbare elektrische Durchführung, insbesondere eine Cermetdurchführung, bestehend aus weniger Einzelteilen bereitzustellen. Eine weitere Aufgabe der Erfindung ist es, eine elektrisch kontaktierbare elektrische Durchführung, insbesondere eine Cermetdurchführung, beinhaltend weniger Verbindungsstellen zwischen verschiedenen Materialien bereitzustellen. Eine weitere Aufgabe der Erfindung ist es, eine elektrisch kontaktierbare elektrische Durchführung, insbesondere eine Cermetdurchführung, welche weniger aufwendig oder in weniger verfahrensschritten oder beides herstellbar ist bereitzustellen. Eine weitere Aufgabe der Erfindung ist es, eine elektrisch kontaktierbare elektrische Durchführung, insbesondere eine Cermetdurchführung, welche eine kompaktere Bauweise aufweist bereitzustellen. Eine weitere Aufgabe der Erfindung ist es, eine elektrisch kontaktierbare elektrische Durchführung, insbesondere eine Cermetdurchführung, welche eine Kombination aus zwei oder mehr der obigen Vorteile aufweist bereitzustellen. Eine weitere Aufgabe der Erfindung ist es, ein implantierbares elektrisches medizinisches Gerät, welches eine elektrische Durchführung mit mindestens einem der obigen Vorteile beinhaltet, bereitzustellen. Eine weitere Aufgabe der Erfindung ist es, ein implantierbares elektrisches medizinisches Gerät mit einer erhöhten Betriebsdauer oder Lebensdauer bereitzustellen. Eine weitere Aufgabe der Erfindung ist es, ein implantierbares elektrisches medizinisches Gerät, welches weniger fehleranfällig im Einsatz in einem menschlichen Körper ist, bereitzustellen. Eine weitere Aufgabe der Erfindung ist es, ein implantierbares elektrisches medizinisches Gerät, welches zuverlässiger in einem menschlichen Körper arbeitet, bereitzustellen.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen.

Einen Beitrag zur Erfüllung mindestens einer der erfindungsgemäßen Aufgaben leistet eine Ausführungsform 1 einer Vorrichtung 1, beinhaltend einen Keramikkörper, beinhaltend eine erste Oberfläche und eine weitere Oberfläche; wobei die erste Oberfläche der weiteren Oberfläche gegenüber liegt; wobei die erste Oberfläche eine erste Öffnung beinhaltet; wobei die weitere Oberfläche eine weitere Öffnung beinhaltet; wobei die erste Öffnung und die weitere Öffnung durch einen Tunnel verbunden sind; wobei der Tunnel eine Tunnelfüllung mindestens teilweise beinhaltet und durch die Tunnelfüllung verschlossen ist; wobei die Tunnelfüllung einen ersten Bestandteil und einen zweiten Bestandteil beinhaltet; wobei der erste Bestandteil ein Cermet beinhaltet; wobei der erste Bestandteil und der zweite Bestandteil elektrisch leitend miteinander verbunden sind; wobei eine der weiteren Öffnung zugewandte Oberfläche der Tunnelfüllung mindestens teilweise eine Oberfläche des zweiten Bestandteils ist; wobei der erste Bestandteil gekennzeichnet ist durch eine erste elektrische Leitfähigkeit; wobei der zweite Bestandteil gekennzeichnet ist durch eine zweite elektrische Leitfähigkeit; wobei der Keramikkörper gekennzeichnet ist durch eine weitere elektrische Leitfähigkeit; wobei die erste elektrische Leitfähigkeit um mindestens 1·10⁵ S/m, bevorzugt um mindestens 5·10⁵ S/m, bevorzugter um mindestens 1·10⁶ S/m, mehr ist als die weitere elektrische Leitfähigkeit; wobei die zweite elektrische Leitfähigkeit sich von der ersten elektrischen Leitfähigkeit um mindestens 5·10⁴ S/m, bevorzugt um mindestens 1·10⁵ S/m, bevorzugter um mindestens 2,5·10⁵ S/m, unterscheidet.

Eine erfindungsgemäße Ausführungsform 2 der Vorrichtung 1 ist nach der Ausführungsform 1 ausgestaltet, wobei die zweite elektrische Leitfähigkeit mehr ist als die erste elektrische Leitfähigkeit.

Eine erfindungsgemäße Ausführungsform 3 der Vorrichtung 1 ist nach der Ausführungsform 1 oder 2 ausgestaltet, wobei der erste Bestandteil einen ersten Metallanteil bezogen auf das Gewicht des ersten Bestandteils hat; wobei der zweite Bestandteil einen zweiten Metallanteil bezogen auf das Gewicht des zweiten Bestandteils hat; wobei der zweite Metallanteil um mindestens 5 Gew.-%, bevorzugt um mindestens 10 Gew.-%, bevorzugter um mindestens 15 Gew.-%, mehr ist als der erste Metallanteil.

Eine erfindungsgemäße Ausführungsform 4 der Vorrichtung 1 ist nach einer der vorhergehenden Ausführungsformen ausgestaltet, wobei die Tunnelfüllung einen dritten Bestandteil beinhaltet; wobei der dritte Bestandteil elektrisch leitend mit dem ersten Bestandteil oder dem zweiten Bestandteil oder beiden verbunden ist; wobei eine der ersten Öffnung zugewandte Oberfläche der Tunnelfüllung mindestens teilweise eine Oberfläche des dritten Bestandteils ist; wobei der dritte Bestandteil gekennzeichnet ist durch eine dritte elektrische Leitfähigkeit; wobei die dritte elektrische Leitfähigkeit sich von der ersten elektrischen Leitfähigkeit um mindestens 5·10⁴ S/m, bevorzugt um mindestens 1·10⁵ S/m, bevorzugter um mindestens 2,5·10⁵ S/m, unterscheidet.

Eine erfindungsgemäße Ausführungsform 5 der Vorrichtung 1 ist nach der Ausführungsform 4 ausgestaltet, wobei die dritte elektrische Leitfähigkeit mehr ist als die erste elektrische Leitfähigkeit.

Eine erfindungsgemäße Ausführungsform 6 der Vorrichtung 1 ist nach der Ausführungsform 4 oder 5 ausgestaltet, wobei der erste Bestandteil einen ersten Metallanteil bezogen auf das Gewicht des ersten Bestandteils hat; wobei der dritte Bestandteil einen dritten Metallanteil bezogen auf das Gewicht des dritten Bestandteils hat; wobei der dritte Metallanteil um mindestens 5 Gew.-%, bevorzugt um mindestens 10 Gew.-%, bevorzugter um mindestens 15 Gew.-%, mehr ist als der erste Metallanteil.

Eine erfindungsgemäße Ausführungsform 7 der Vorrichtung 1 ist nach einer der vorhergehenden Ausführungsformen ausgestaltet, wobei der Tunnel den ersten Bestandteil und den zweiten Bestandteil, oder den ersten Bestandteil und den zweiten Bestandteil und den dritten Bestandteil vollständig beinhaltet. Das heißt die jeweiligen Bestandteile ragen nicht aus der ersten Öffnung oder der weiteren Öffnung oder beiden heraus.

Eine erfindungsgemäße Ausführungsform 8 der Vorrichtung 1 ist nach einer der vorhergehenden Ausführungsformen ausgestaltet, wobei die der weiteren Öffnung zugewandte Oberfläche der Tunnelfüllung einen Abstand von weniger als 3 mm, bevorzugt von weniger als 2 mm, bevorzugter von weniger als 1 mm, bevorzugter von weniger als 800 µm, bevorzugter von weniger als 600 µm, bevorzugter von weniger als 400 µm, am bevorzugtesten von weniger als 200 µm, von der weiteren Öffnung hat. Bevorzugt hat die der weiteren Öffnung zugewandte Oberfläche der Tunnelfüllung den Abstand von der weiteren Öffnung in Richtung zu der ersten Öffnung, also in Richtung in den Tunnel hinein.

Eine erfindungsgemäße Ausführungsform 9 der Vorrichtung 1 ist nach einer der Ausführungsformen 4 bis 8 ausgestaltet, wobei die der ersten Öffnung zugewandte Oberfläche der Tunnelfüllung einen Abstand von weniger als 3 mm, bevorzugt von weniger als 2 mm, bevorzugter von weniger als 1 mm, bevorzugter von weniger als 800 µm, bevorzugter von weniger als 600 µm, bevorzugter von weniger als 400 µm, am bevorzugtesten von weniger als 200 µm, von der ersten Öffnung hat. Bevorzugt hat die der ersten Öffnung zugewandte Oberfläche der Tunnelfüllung den Abstand von der ersten Öffnung in Richtung zu der weiteren Öffnung, also in Richtung in den Tunnel hinein.

Eine erfindungsgemäße Ausführungsform 10 der Vorrichtung 1 ist nach einer der vorhergehenden Ausführungsformen ausgestaltet, wobei die zweite elektrische Leitfähigkeit oder die dritte elektrische Leitfähigkeit oder beide in einem Bereich von 1·10³ bis 1·10⁷ S/m, bevorzugt von 1·10⁴ bis 1·10⁷ S/m, bevorzugter von 1·10⁶ bis 1·10⁷ S/m, liegen.

Eine erfindungsgemäße Ausführungsform 11 der Vorrichtung 1 ist nach einer der vorhergehenden Ausführungsformen ausgestaltet, wobei der zweite Bestandteil oder der dritte Bestandteil oder beide mit einem elektrischen Leiter verbunden sind. Ein bevorzugter elektrischer Leiter ist ein Draht oder ein Lot oder beides. Bevorzugt ist der elektrische Leiter mit dem zweiten Bestandteil oder dem dritten Bestandteil oder beiden durch eines ausgewählt aus der Gruppen bestehend aus einem Bonden, einem Schweißen, und einem Löten, oder einer Kombination aus mindestens zwei davon verbunden. Bevorzugt ist der elektrische Leiter nicht von dem Tunnel beinhaltet.

Eine erfindungsgemäße Ausführungsform 12 der Vorrichtung 1 ist nach einer der vorhergehenden Ausführungsformen ausgestaltet, wobei eines ausgewählt aus der Gruppe bestehend aus dem ersten Bestandteil, dem zweiten Bestandteil und dem dritten Bestandteil, oder eine Kombination aus mindestens zwei davon ein Cermet ist.

Eine erfindungsgemäße Ausführungsform 13 der Vorrichtung 1 ist nach einer der vorhergehenden Ausführungsformen ausgestaltet, wobei der erste Bestandteil eine erste Bestandteilschicht bildet; wobei der zweite Bestandteil eine zweite Bestandteilschicht bildet; wobei die zweite Bestandteilschicht die erste Bestandteilschicht überlagert.

Eine erfindungsgemäße Ausführungsform 14 der Vorrichtung 1 ist nach einer der vorhergehenden Ausführungsformen ausgestaltet, wobei der erste Bestandteil eine konkave erste Bestandteiloberfläche beinhaltet; wobei der zweite Bestandteil eine konvexe zweite Bestandteiloberfläche beinhaltet, wobei die konvexe zweite Bestandteiloberfläche die konkave erste Bestandteiloberfläche überlagert. In diesem Zusammenhang ist auch eine kegelartige Ausgestaltung des zweiten Bestandteils möglich. In diesem Fall ist die zweite Bestandteiloberfläche eine Kegelmantelfläche und die erste Bestandteiloberfläche ist invers dazu, bevorzugt trichterförmig.

Eine erfindungsgemäße Ausführungsform 15 der Vorrichtung 1 ist nach einer der Ausführungsformen 1 bis 13 ausgestaltet, wobei der erste Bestandteil eine konvexe erste Bestandteiloberfläche beinhaltet; wobei der zweite Bestandteil eine konkave zweite Bestandteiloberfläche beinhaltet; wobei die konkave zweite Bestandteiloberfläche die konvexe erste Bestandteiloberfläche überlagert. In diesem Zusammenhang ist auch eine Ausgestaltung der ersten Bestandteiloberfläche als Kegelmantelfläche möglich. In diesem Fall ist die zweite Bestandteiloberfläche invers dazu, bevorzugt trichterförmig.

Eine erfindungsgemäße Ausführungsform 16 der Vorrichtung 1 ist nach einer der Ausführungsformen 3 bis 15 ausgestaltet, wobei der erste Bestandteil eine erste Bestandteilschicht bildet; wobei der dritte Bestandteil eine dritte Bestandteilschicht bildet; wobei die erste Bestandteilschicht die dritte Bestandteilschicht überlagert.

Eine erfindungsgemäße Ausführungsform 17 der Vorrichtung 1 ist nach einer der Ausführungsformen 3 bis 16 ausgestaltet, wobei der erste Bestandteil eine konkave weitere erste Bestandteiloberfläche beinhaltet; wobei der dritte Bestandteil eine konvexe dritte Bestandteiloberfläche beinhaltet; wobei die konkave weitere erste Bestandteiloberfläche die konvexe dritte Bestandteiloberfläche überlagert. In diesem Zusammenhang ist auch eine kegelartige Ausgestaltung des dritten Bestandteils möglich. In diesem Fall ist die dritte Bestandteiloberfläche eine Kegelmantelfläche und die weitere erste Bestandteiloberfläche ist invers dazu, bevorzugt trichterförmig.

Eine erfindungsgemäße Ausführungsform 18 der Vorrichtung 1 ist nach einer der Ausführungsformen 3 bis 16 ausgestaltet, wobei der erste Bestandteil eine konvexe weitere erste Bestandteiloberfläche beinhaltet; wobei der dritte Bestandteil eine konkave dritte Bestandteiloberfläche beinhaltet; wobei die konvexe weitere erste Bestandteiloberfläche die konkave dritte Bestandteiloberfläche überlagert. In diesem Zusammenhang ist auch eine Ausgestaltung der weiteren ersten Bestandteiloberfläche als Kegelmantelfläche möglich. In diesem Fall ist die dritte Bestandteiloberfläche invers dazu, bevorzugt trichterförmig.

Eine erfindungsgemäße Ausführungsform 19 der Vorrichtung 1 ist nach einer der vorhergehenden Ausführungsformen ausgestaltet, wobei der Keramikkörper und der erste Bestandteile und der zweite Bestandteil, oder der Keramikkörper und der erste Bestandteile und der zweite Bestandteil und der dritte Bestandteil als ein Stück ausgebildet sind. Bevorzugt sind der Keramikkörper und der erste Bestandteil und der zweite Bestandteil, oder der Keramikkörper und der erste Bestandteile und der zweite Bestandteil und der dritte Bestandteil aus einem einzigen Grünkörper gesintert.

Ferner offenbart wird eine Ausführungsform 1 einer Vorrichtung 2, beinhaltend eine erste Keramikvorläuferschicht und eine zweite Keramikvorläuferschicht,
a) wobei für die erste Keramikvorläuferschicht mindestens gilt:
   i) die erste Keramikvorläuferschicht beinhaltet eine erste Schichtoberfläche und eine weitere Schichtoberfläche,
   ii) die erste Schichtoberfläche liegt der weiteren Schichtoberfläche gegenüber,
   iii) die erste Schichtoberfläche beinhaltet eine erste Öffnung,
   iv) die weitere Schichtoberfläche beinhaltet eine weitere Öffnung,
   v) die erste Öffnung und die weitere Öffnung sind durch einen ersten Tunnel verbunden,
   vi) der erste Tunnel beinhaltet eine erste Tunnelfüllung mindestens teilweise und ist durch die erste Tunnelfüllung verschlossen,
   vii) die erste Tunnelfüllung beinhaltet eine erste Zusammensetzung und eine zweite Zusammensetzung,
   viii) eine der weiteren Öffnung zugewandte Oberfläche der ersten Tunnelfüllung ist mindestens teilweise eine Oberfläche der zweiten Zusammensetzung,
   ix) die erste Zusammensetzung hat einen ersten Zusammensetzungsmetallanteil bezogen auf das Gewicht der ersten Zusammensetzung,
   x) die zweite Zusammensetzung hat einen zweiten Zusammensetzungsmetallanteil bezogen auf das Gewicht der zweiten Zusammensetzung, und
   xi) der zweite Zusammensetzungsmetallanteil unterscheidet sich um mindestens 3 Gew.-%, bevorzugt um mindestens 7 Gew.-%, bevorzugter um mindestens 15 Gew.-%, von dem ersten Zusammensetzungsmetallanteil,
b) wobei für die zweite Keramikvorläuferschicht mindestens gilt:
   i) die zweite Keramikvorläuferschicht beinhaltet eine erste Schichtoberfläche und eine weitere Schichtoberfläche,
   ii) die erste Schichtoberfläche liegt der weiteren Schichtoberfläche gegenüber,
   iii) die erste Schichtoberfläche beinhaltet eine erste Öffnung,
   iv) die weitere Schichtoberfläche beinhaltet eine weitere Öffnung,
   v) die erste Öffnung und die weitere Öffnung sind durch einen zweiten Tunnel verbunden,
   vi) der zweite Tunnel beinhaltet eine zweite Tunnelfüllung mindestens teilweise und ist durch die zweite Tunnelfüllung verschlossen,
   vii)die zweite Tunnelfüllung beinhaltet eine zweite erste Zusammensetzung,
   viii) die zweite erste Zusammensetzung hat einen zweiten ersten Zusammensetzungsmetallanteil bezogen auf das Gewicht der zweiten ersten Zusammensetzung, und
   ix) der zweite Zusammensetzungsmetallanteil unterscheidet sich um mindestens 3 Gew.-%, bevorzugt um mindestens 7 Gew.-%, bevorzugter um mindestens 15 Gew.-%, von dem zweiten ersten Zusammensetzungsmetallanteil,
c) wobei die weitere Schichtoberfläche der zweiten Keramikvorläuferschicht mit der ersten Schichtoberfläche der ersten Keramikvorläuferschicht so kontaktiert ist, dass die erste Tunnelfüllung und die zweite Tunnelfüllung kontaktiert sind. Bevorzugt ist die erste Keramikvorläuferschicht auf die zweite Keramikvorläuferschicht oder umgekehrt oder beides laminiert.

Eine Ausführungsform 2 der Vorrichtung 2 ist nach der Ausführungsform 1 ausgestaltet, wobei die Vorrichtung weiter eine dritte Keramikvorläuferschicht beinhaltet, wobei die dritte Keramikvorläuferschicht eine erste Schichtoberfläche und eine weitere Schichtoberfläche beinhaltet, wobei die erste Schichtoberfläche der weiteren Schichtoberfläche gegenüber liegt, wobei die erste Schichtoberfläche eine erste Öffnung beinhaltet, wobei die weitere Schichtoberfläche eine weitere Öffnung beinhaltet, wobei die erste Öffnung und die weitere Öffnung durch einen dritten Tunnel verbunden sind, wobei der dritte Tunnel eine dritte Tunnelfüllung mindestens teilweise beinhaltet und durch die dritte Tunnelfüllung verschlossen ist, wobei die dritte Tunnelfüllung eine dritte erste Zusammensetzung und eine weitere zweite Zusammensetzung beinhaltet, wobei eine der ersten Öffnung zugewandte Oberfläche der dritten Tunnelfüllung mindestens teilweise eine Oberfläche der weiteren zweiten Zusammensetzung ist, wobei die dritte erste Zusammensetzung einen dritten ersten Zusammensetzungsmetallanteil bezogen auf das Gewicht der dritten ersten Zusammensetzung hat, wobei die weitere zweite Zusammensetzung einen weiteren zweiten Zusammensetzungsmetallanteil bezogen auf das Gewicht der weiteren zweiten Zusammensetzung hat, wobei sich der weitere zweite Zusammensetzungsmetallanteil um mindestens 3 Gew.-%, bevorzugt um mindestens 7 Gew.-%, bevorzugter um mindestens 15 Gew.-%, von dem dritten ersten Zusammensetzungsmetallanteil unterscheidet, wobei die erste Schichtoberfläche der zweiten Keramikvorläuferschicht durch die weitere Schichtoberfläche der dritten Keramikvorläuferschicht überlagert wird.

Offenbart wird weiter eine Ausführungsform 1 einer Vorrichtung 3 beinhaltend eine Keramikvorläuferschicht, wobei für die Keramikvorläuferschicht mindestens gilt:
a) die Keramikvorläuferschicht beinhaltet eine erste Schichtoberfläche und eine weitere Schichtoberfläche,
b) die erste Schichtoberfläche liegt der weiteren Schichtoberfläche gegenüber,
c) die erste Schichtoberfläche beinhaltet eine erste Öffnung,
d) die weitere Schichtoberfläche beinhaltet eine weitere Öffnung,
e) die erste Öffnung und die weitere Öffnung sind durch einen Tunnel verbunden,
f) der Tunnel beinhaltet eine Tunnelfüllung mindestens teilweise und ist durch die Tunnelfüllung verschlossen,
g) die Tunnelfüllung beinhaltet eine erste Zusammensetzung, eine zweite Zusammensetzung und eine dritte Zusammensetzung,
h) eine der weiteren Öffnung zugewandte Oberfläche der Tunnelfüllung ist mindestens teilweise eine Oberfläche der zweiten Zusammensetzung,
i) eine der ersten Öffnung zugewandte Oberfläche der Tunnelfüllung ist mindestens teilweise eine Oberfläche der dritten Zusammensetzung,
j) die erste Zusammensetzung hat einen ersten Zusammensetzungsmetallanteil bezogen auf das Gewicht der ersten Zusammensetzung,
k) die zweite Zusammensetzung hat einen zweiten Zusammensetzungsmetallanteil bezogen auf das Gewicht der zweiten Zusammensetzung,
l) die dritte Zusammensetzung hat einen dritten Zusammensetzungsmetallanteil bezogen aus das Gewicht der dritten Zusammensetzung, und
m) der zweite Zusammensetzungsmetallanteil oder der dritte Zusammensetzungsmetallanteil oder beide, bevorzugt beide, unterscheidet sich um mindestens 3 Gew.-%, bevorzugt um mindestens 7 Gew.-%, bevorzugter um mindestens 15 Gew.-%, von dem ersten Zusammensetzungsmetallanteil.

Eine Ausführungsform 3 der Vorrichtung 2 ist nach der Ausführungsform 1 oder 2 ausgestaltet und eine erfindungsgemäße Ausführungsform 2 der Vorrichtung 3 ist nach der Ausführungsform 1 ausgestaltet, wobei eines ausgewählt aus der Gruppe bestehend aus der ersten Zusammensetzung, der zweiten ersten Zusammensetzung, der dritten ersten Zusammensetzung, der zweiten Zusammensetzung, der weiteren zweiten Zusammensetzung, und der dritten Zusammensetzung, oder eine Kombination aus mindestens zwei davon eine Cermetpaste ist.

Offenbart wird ferner eine Ausführungsform 1 eines Verfahrens 1, beinhaltend als Verfahrensschritte
a) Bereitstellen einer ersten Keramikvorläuferschicht, beinhaltend eine erste Schichtoberfläche und eine weitere Schichtoberfläche, wobei die erste Schichtoberfläche der weiteren Schichtoberfläche gegenüber liegt, wobei die erste Schichtoberfläche eine erste Öffnung beinhaltet, wobei die weitere Schichtoberfläche eine weitere Öffnung beinhaltet, wobei die erste Öffnung und die weitere Öffnung durch einen ersten Tunnel verbunden sind, wobei der erste Tunnel eine erste Tunnelfüllung mindestens teilweise beinhaltet und durch die erste Tunnelfüllung verschlossen ist, wobei die erste Tunnelfüllung eine erste Zusammensetzung und eine zweite Zusammensetzung beinhaltet, wobei eine der weiteren Öffnung zugewandte Oberfläche der ersten Tunnelfüllung mindestens teilweise eine Oberfläche der zweiten Zusammensetzung ist, wobei die erste Zusammensetzung einen ersten Zusammensetzungsmetallanteil bezogen auf das Gewicht der ersten Zusammensetzung hat, wobei die zweite Zusammensetzung einen zweiten Zusammensetzungsmetallanteil bezogen auf das Gewicht der zweiten Zusammensetzung hat, wobei der zweite Zusammensetzungsmetallanteil sich um mindestens 3 Gew.-%, bevorzugt um mindestens 7 Gew.-%, bevorzugter um mindestens 15 Gew.-%, von dem ersten Zusammensetzungsmetallanteil unterscheidet;
b) Bereitstellen einer zweiten Keramikvorläuferschicht, beinhaltend eine erste Schichtoberfläche und eine weitere Schichtoberfläche, wobei die erste Schichtoberfläche der weiteren Schichtoberfläche gegenüber liegt, wobei die erste Schichtoberfläche eine erste Öffnung beinhaltet, wobei die weitere Schichtoberfläche eine weitere Öffnung beinhaltet, wobei die erste Öffnung und die weitere Öffnung durch einen zweiten Tunnel verbunden sind, wobei der zweite Tunnel eine zweite Tunnelfüllung mindestens teilweise beinhaltet und durch die zweite Tunnelfüllung verschlossen ist, wobei die zweite Tunnelfüllung eine zweite erste Zusammensetzung beinhaltet, wobei die zweite erste Zusammensetzung einen zweiten ersten Zusammensetzungsmetallanteil bezogen auf das Gewicht der zweiten ersten Zusammensetzung hat, wobei der zweite Zusammensetzungsmetallanteil sich um mindestens 3 Gew.-%, bevorzugt um mindestens 7 Gew.-%, bevorzugter um mindestens 15 Gew.-%, von dem zweiten ersten Zusammensetzungsmetallanteil unterscheidet; und
c) Kontaktieren der weiteren Schichtoberfläche der zweiten Keramikvorläuferschicht mit der ersten Schichtoberfläche der ersten Keramikvorläuferschicht, so dass die erste Tunnelfüllung und die zweite Tunnelfüllung kontaktiert werden.

Eine Ausführungsform 2 des Verfahrens 1 ist nach der Ausführungsform 1 ausgestaltet, wobei vor Verfahrensschritt c) eine dritte Keramikvorläuferschicht bereitgestellt wird, wobei die dritte Keramikvorläuferschicht eine erste Schichtoberfläche und eine weitere Schichtoberfläche beinhaltet, wobei die erste Schichtoberfläche der weiteren Schichtoberfläche gegenüber liegt, wobei die erste Schichtoberfläche eine erste Öffnung beinhaltet, wobei die weitere Schichtoberfläche eine weitere Öffnung beinhaltet, wobei die erste Öffnung und die weitere Öffnung durch einen dritten Tunnel verbunden sind, wobei der dritte Tunnel eine dritte Tunnelfüllung mindestens teilweise beinhaltet und durch die dritte Tunnelfüllung verschlossen ist, wobei die dritte Tunnelfüllung eine dritte erste Zusammensetzung und eine weitere zweite Zusammensetzung beinhaltet, wobei eine der ersten Öffnung zugewandte Oberfläche der dritten Tunnelfüllung eine Oberfläche der weiteren zweiten Zusammensetzung ist, wobei die dritte erste Zusammensetzung einen dritten ersten Zusammensetzungsmetallanteil bezogen auf das Gewicht der dritten ersten Zusammensetzung hat, wobei die weitere zweite Zusammensetzung einen weiteren zweiten Zusammensetzungsmetallanteil bezogen auf das Gewicht der weiteren zweiten Zusammensetzung hat, wobei der weitere zweite Zusammensetzungsmetallanteil sich um mindestens 3 Gew.-%, bevorzugt um mindestens 7 Gew.-%, bevorzugter um mindestens 15 Gew.-%, von dem dritten ersten Zusammensetzungsmetallanteil unterscheidet, wobei in Verfahrensschritt c) die erste Schichtoberfläche der zweiten Keramikvorläuferschicht durch die weitere Schichtoberfläche der dritten Keramikvorläuferschicht überlagert wird.

Eine Ausführungsform 3 des Verfahrens 1 ist nach der Ausführungsform 1 oder 2 ausgestaltet, wobei in Verfahrensschritt c) die erste Schichtoberfläche der zweiten Keramikvorläuferschicht durch mindestens eine weitere Keramikvorläuferschicht so überlagert wird, dass die mindestens eine weitere Keramikvorläuferschicht zwischen der zweiten Keramikvorläuferschicht und der dritten Keramikvorläuferschicht positioniert wird.

Eine Ausführungsform 4 des Verfahrens 1 ist nach einer der Ausführungsformen 1 bis 3 ausgestaltet, wobei in Verfahrensschritt c) die weitere Schichtoberfläche der zweiten Keramikvorläuferschicht mit der ersten Schichtoberfläche der ersten Keramikvorläuferschicht durch ein isostatisches Pressen verbunden wird. Bevorzugt erfolgt das isostatische Pressen im Zuge eines Laminierens.

Eine Ausführungsform 5 des Verfahrens 1 ist nach einer der Ausführungsformen 1 bis 4 ausgestaltet, wobei in Verfahrensschritt a) das Bereitstellen der ersten Keramikvorläuferschicht beinhaltet
i) Bereitstellen einer ungefüllten ersten Keramikvorläuferschicht, beinhaltend die erste Schichtoberfläche und die weitere Schichtoberfläche, wobei die erste Schichtoberfläche der weiteren Schichtoberfläche gegenüber liegt, wobei die erste Schichtoberfläche die erste Öffnung beinhaltet, wobei die weitere Schichtoberfläche die weitere Öffnung beinhaltet, wobei die erste Öffnung und die weitere Öffnung durch den ersten Tunnel verbunden sind;
ii) Bereitstellen der ersten Zusammensetzung;
iii) Einbringen der ersten Zusammensetzung durch die weitere Öffnung in den ersten Tunnel in mindestens einem Einbringschritt, wobei nach jedem Einbringschritt eine eingebrachte Portion der ersten Zusammensetzung getrocknet wird;
iv) Bereitstellen der zweiten Zusammensetzung; und
v) Überlagern der ersten Zusammensetzung in dem ersten Tunnel mit der zweiten Zusammensetzung in mindestens einem Einbringschritt,
   wobei nach jedem Einbringschritt eine eingebrachte Portion der zweiten Zusammensetzung getrocknet wird.

Bevorzugt wird in Verfahrensschritt v) die erste Zusammensetzung in so vielen Einbringschritten mit so vielen Portionen der zweiten Zusammensetzung überlagert wie nötig sind, um den ersten Tunnel vollständig zu füllen und die weitere Öffnung bündig zu der weiteren Schichtoberfläche zu verschließen.

Eine Ausführungsform 6 des Verfahrens 1 ist nach der Ausführungsform 5 ausgestaltet, wobei in Verfahrensschritt iii) die erste Zusammensetzung in mindestens einem ersten Einbringschritt und einem weiteren Einbringschritt durch die weitere Öffnung in den ersten Tunnel eingebracht wird, wobei in dem ersten Einbringschritt zunächst eine erste Portion der ersten Zusammensetzung eingebracht wird und danach in dem weiteren Einbringschritt eine weitere Portion der ersten Zusammensetzung eingebracht wird, wobei die erste Portion der ersten Zusammensetzung eine erste Viskosität hat, wobei die weitere Portion der ersten Zusammensetzung eine weitere Viskosität hat, wobei die weitere Viskosität mehr ist als die erste Viskosität.

Bevorzugt ist die weitere Viskosität hierbei um 40 Pa·s, bevorzugt um 50 Pa·s, bevorzugter um 60 Pa·s, mehr als die erste Viskosität. Bevorzugt hat die erste Portion einen ersten Feststoffanteil und die weitere Portion einen weiteren Feststoffanteil, wobei der weitere Feststoffanteil mehr ist als der erste Feststoffanteil. Bevorzugt ist der weitere Feststoffanteil hierbei um mindestens 10 Gew.-%, bevorzugt um mindestens 15 Gew.-%, bevorzugter um mindestens 20 Gew.-%, mehr als der erste Feststoffanteil. Ein bevorzugter Feststoff ist hierbei ein Pulver. Ein bevorzugtes Pulver ist ein Keramikpulver oder ein Metallpulver oder beides. In diesem Zusammenhang wird eine Wand des ersten Tunnels bevorzugt zunächst mit einer dünnerflüssigen Paste benetzt und anschließend eine im Vergleich zur dünnerflüssigen Paste dickerflüssige Paste in den ersten Tunnel eingebracht. Bevorzugt entsteht so in dem ersten Tunnel in radialer Richtung eine Schichtung aus dünnerflüssiger und dickerflüssiger Paste, wobei die Dünnerflüssige Paste die Tunnelwand benetzt. Dies verbessert bevorzugt eine Verbindung zwischen der Tunnelwand und der eingebrachten Paste und/oder dem durch Brennen daraus entstehenden ersten Bestandteil.

Eine Ausführungsform 7 des Verfahrens 1 ist nach einer der Ausführungsformen 1 bis 4 ausgestaltet, wobei in Verfahrensschritt a) das Bereitstellen der ersten Keramikvorläuferschicht beinhaltet
i) Bereitstellen einer ungefüllten ersten Keramikvorläuferschicht, beinhaltend die erste Schichtoberfläche und die weitere Schichtoberfläche, wobei die erste Schichtoberfläche der weiteren Schichtoberfläche gegenüber liegt, wobei die erste Schichtoberfläche die erste Öffnung beinhaltet, wobei die weitere Schichtoberfläche die weitere Öffnung beinhaltet, wobei die erste Öffnung und die weitere Öffnung durch den ersten Tunnel verbunden sind;
ii) Bereitstellen der zweiten Zusammensetzung;
iii) Einbringen der zweiten Zusammensetzung durch die erste Öffnung in den ersten Tunnel in mindestens einem Einbringschritt, wobei nach jedem Einbringschritt eine eingebrachte Portion der zweiten Zusammensetzung getrocknet wird;
iv) Bereitstellen der ersten Zusammensetzung;
v) Überlagern der zweiten Zusammensetzung in dem ersten Tunnel mit der ersten Zusammensetzung in mindestens einem Einbringschritt, wobei nach jedem Einbringschritt eine eingebrachte Portion der ersten Zusammensetzung getrocknet wird.

Bevorzugt wird in Verfahrensschritt v) die zweite Zusammensetzung in so vielen Einbringschritten mit so vielen Portionen der ersten Zusammensetzung überlagert wie nötig sind, um den ersten Tunnel vollständig zu füllen und die erste Öffnung bündig zu der weiteren Schichtoberfläche zu verschließen.

Eine Ausführungsform 8 des Verfahrens 1 ist nach der Ausführungsform 7 ausgestaltet, wobei in Verfahrensschritt iii) die zweite Zusammensetzung in mindestens einem ersten Einbringschritt und einem weiteren Einbringschritt durch die erste Öffnung in den ersten Tunnel eingebracht wird, wobei in dem ersten Einbringschritt zunächst eine erste Portion der zweiten Zusammensetzung eingebracht wird und danach in dem weiteren Einbringschritt eine weitere Portion der zweiten Zusammensetzung eingebracht wird, wobei die erste Portion der zweiten Zusammensetzung eine zweite erste Viskosität hat, wobei die weitere Portion der ersten Zusammensetzung eine zweite weitere Viskosität hat, wobei die zweite weitere Viskosität mehr ist als die zweite erste Viskosität.

Bevorzugt ist die zweite weitere Viskosität hierbei um 40 Pa·s, bevorzugt um 50 Pa·s , bevorzugter um 60 Pa·s, mehr als die zweite erste Viskosität. Bevorzugt hat die erste Portion einen zweiten ersten Feststoffanteil und die weitere Portion einen zweiten weiteren Feststoffanteil, wobei der zweite weitere Feststoffanteil mehr ist als der zweite erste Feststoffanteil. Bevorzugt ist der zweite weitere Feststoffanteil hierbei um mindestens 10 Gew.-%, bevorzugt um mindestens 15 Gew.-%, bevorzugter um mindestens 20 Gew.-%, mehr als der zweite erste Feststoffanteil. Ein bevorzugter Feststoff ist hierbei ein Pulver. Ein bevorzugtes Pulver ist ein Keramikpulver oder ein Metallpulver oder beides. In diesem Zusammenhang wird eine Wand des ersten Tunnels bevorzugt zunächst mit einer dünnerflüssigen Paste benetzt und anschließend eine im Vergleich zur dünnerflüssigen Paste dickerflüssige Paste in den ersten Tunnel eingebracht. Bevorzugt entsteht so in dem ersten Tunnel in radialer Richtung eine Schichtung aus dünnerflüssiger und dickerflüssiger Paste, wobei die dünnerflüssige Paste die Tunnelwand benetzt. Dies verbessert bevorzugt eine Verbindung zwischen der Tunnelwand und der eingebrachten Paste und/oder dem durch Brennen daraus entstehenden zweiten Bestandteil.

Eine Ausführungsform 9 des Verfahrens 1 ist nach einer der Ausführungsformen 1 bis 8 ausgestaltet, wobei das Trocknen der eingebrachten Portion ein Erwärmen der eingebrachten Portion auf eine Trockentemperatur in einem Bereich von 30 bis 500°C, bevorzugt von 40 bis 400°C, bevorzugter von 45 bis 300°C, bevorzugter von 50 bis 200°C, bevorzugter von 55 bis 100°C, bevorzugter von 60 bis 95°C, bevorzugter von 70 bis 90°C, am bevorzugtesten von 75 bis 85°C, beinhaltet.

Eine Ausführungsform 10 des Verfahrens 1 ist nach einer der Ausführungsformen 1 bis 9 ausgestaltet, wobei das Verfahren nach Verfahrensschritt c) als weiteren Verfahrensschritt beinhaltet
d) Brennen der ersten Keramikvorläuferschicht und der zweiten Keramikvorläuferschicht, oder der ersten Keramikvorläuferschicht und der zweiten Keramikvorläuferschicht und der dritten Keramikvorläuferschicht, oder der ersten Keramikvorläuferschicht und der zweiten Keramikvorläuferschicht und der dritten Keramikvorläuferschicht und der mindestens einen weiteren Keramikvorläuferschicht.
Bevorzugt beinhaltet die erste Keramikvorläuferschicht n erfindungsgemäße erste Tunnel, wobei jeder erste Tunnel in dem erfindungsgemäßen Verfahren gefüllt wird wie in den obigen Ausführungsformen für den ersten Tunnel beschrieben, wobei n eine natürliche Zahl größer 0, bevorzugt größer 2, bevorzugt größer 7 bevorzugt größer 15, bevorzugter größer 31, bevorzugter größer 63, bevorzugter größer 127, bevorzugter größer 255, am bevorzugtesten größer 511 ist. Bevorzugt beinhaltet die zweite Keramikvorläuferschicht n erfindungsgemäße zweite Tunnel, wobei jeder zweite Tunnel in dem erfindungsgemäßen Verfahren gefüllt wird wie in den obigen Ausführungsformen für den zweiten Tunnel beschrieben, wobei n eine natürliche Zahl größer 0, bevorzugt größer 2, bevorzugt größer 7 bevorzugt größer 15, bevorzugter größer 31, bevorzugter größer 63, bevorzugter größer 127, bevorzugter größer 255, am bevorzugtesten größer 511 ist. Bevorzugt beinhaltet die dritte Keramikvorläuferschicht n erfindungsgemäße dritte Tunnel, wobei jeder dritte Tunnel in dem erfindungsgemäßen Verfahren gefüllt wird wie in den obigen Ausführungsformen für den dritten Tunnel beschrieben, wobei n eine natürliche Zahl größer 0, bevorzugt größer 2, bevorzugt größer 7 bevorzugt größer 15, bevorzugter größer 31, bevorzugter größer 63, bevorzugter größer 127, bevorzugter größer 255, am bevorzugtesten größer 511 ist. In einem weitern Aspekt dieser Ausgestaltung ist es möglich, dass die miteinander kontaktierten Keramikvorläuferschichten vor dem Brennen in Verfahrensschritt d) so zerteilt werden, dass die Zahl n der Tunnel pro Keramikvorläuferschicht mindestens um einen Faktor 2, bevorzugt mindestens um einen Faktor 3, bevorzugter mindestens um einen Faktor 4, am bevorzugtesten mindestens um einen Faktor 5, verringert wird.

Eine Ausführungsform 11 des Verfahrens 1 ist nach der Ausführungsform 10 ausgestaltet, wobei in Verfahrensschritt d)
A) bei dem Brennen der ersten Keramikvorläuferschicht aus der zweiten Zusammensetzung ein zweiter Bestandteil erhalten wird, wobei in einem weiteren Verfahrensschritt der zweite Bestandteil mit einem elektrischen Leiter elektrisch leitend verbunden wird; oder
B) bei dem Brennen der dritten Keramikvorläuferschicht aus der weiteren zweiten Zusammensetzung ein dritter Bestandteil erhalten wird, wobei in einem weiteren Verfahrensschritt der dritte Bestandteil mit einem elektrischen Leiter elektrisch leitend verbunden wird; oder
C) beides.
Ein bevorzugter elektrischer Leiter ist ein Draht oder ein Lot oder beides. Bevorzugt wird der elektrische Leiter mit dem zweiten Bestandteil durch eines ausgewählt aus der Gruppe bestehend aus einem Bonden, einem Schweißen, und einem Löten, oder einer Kombination aus mindestens zwei davon verbunden.

Weiter offenbart wird eine Ausführungsform 1 eines Verfahrens 2, beinhaltend als Verfahrensschritte:
a) Bereitstellen einer ungefüllten Keramikvorläuferschicht, beinhaltend eine erste Schichtoberfläche und eine weitere Schichtoberfläche,
   wobei die erste Schichtoberfläche der weiteren Schichtoberfläche gegenüber liegt, wobei die erste Schichtoberfläche eine erste Öffnung beinhaltet,
   wobei die weitere Schichtoberfläche eine weitere Öffnung beinhaltet,
   wobei die erste Öffnung und die weitere Öffnung durch einen Tunnel verbunden sind;
b) Bereitstellen einer dritten Zusammensetzung,
   wobei die dritte Zusammensetzung einen dritten Zusammensetzungsmetallanteil bezogen auf das Gewicht der dritten Zusammensetzung hat;
c) Bereitstellen einer ersten Zusammensetzung,
   wobei die erste Zusammensetzung einen ersten Zusammensetzungsmetallanteil bezogen auf das Gewicht der ersten Zusammensetzung hat,
   wobei der dritte Zusammensetzungsmetallanteil sich um mindestens 3 Gew.-%, bevorzugt um mindestens 7 Gew.-%, bevorzugter um mindestens 15 Gew.-%, von dem ersten Zusammensetzungsmetallanteil unterscheidet;
d) Bereitstellen einer zweiten Zusammensetzung,
   wobei die zweite Zusammensetzung einen zweiten Zusammensetzungsmetallanteil bezogen auf das Gewicht der zweiten Zusammensetzung hat,
   wobei der zweite Zusammensetzungsmetallanteil sich um mindestens 3 Gew.-%, bevorzugt um mindestens 7 Gew.-%, bevorzugter um mindestens 15 Gew.-%, von dem ersten Zusammensetzungsmetallanteil unterscheidet;
e) Einbringen der dritten Zusammensetzung durch die erste Öffnung in den Tunnel in mindestens einem Einbringschritt,
   wobei nach jedem Einbringschritt eine eingebrachte Portion der dritten Zusammensetzung getrocknet wird;
f) Überlagern der dritten Zusammensetzung in dem Tunnel mit der ersten Zusammensetzung in mindestens einem Einbringschritt,
   wobei die erste Zusammensetzung durch die erste Öffnung in den Tunnel eingebracht wird,
   wobei nach jedem Einbringschritt eine eingebrachte Portion der ersten Zusammensetzung getrocknet wird;
g) Überlagern der ersten Zusammensetzung in dem Tunnel mit der zweiten Zusammensetzung in mindestens einem Einbringschritt,
   wobei die zweite Zusammensetzung durch die erste Öffnung in den Tunnel eingebracht wird,
   wobei nach jedem Einbringschritt eine eingebrachte Portion der zweiten Zusammensetzung getrocknet wird; und

Brennen der Keramikvorläuferschicht, beinhaltend die erste Zusammensetzung, die zweite Zusammensetzung und die dritte Zusammensetzung.

Ein bevorzugtes Trocknen einer eingebrachten Portion beinhaltet ein Erwärmen der eingebrachten Portion auf eine Trockentemperatur in einem Bereich von 30 bis 500°C, bevorzugt von 40 bis 400°C, bevorzugter von 45 bis 300°C, bevorzugter von 50 bis 200°C, bevorzugter von 55 bis 100°C, bevorzugter von 60 bis 95°C, bevorzugter von 70 bis 90°C, am bevorzugtesten von 75 bis 85°C.

Eine Ausführungsform 12 des Verfahrens 1 ist nach einer der Ausführungsformen 1 bis 11 ausgestaltet und eine Ausführungsform 2 des Verfahrens 2 ist nach der Ausführungsform 1 ausgestaltet, wobei eines ausgewählt aus der Gruppe bestehend aus der ersten Zusammensetzung, der zweiten ersten Zusammensetzung, der dritten ersten Zusammensetzung, der zweiten Zusammensetzung, und der weiteren zweiten Zusammensetzung, oder eine Kombination aus mindestens zwei davon eine Cermetpaste ist.

Offenbart wird außerdem eine Ausführungsform 1 eines Keramikvorläufers 1, erhältlich durch das Verfahren 1 nach einer der Ausführungsformen 1 bis 12 oder durch das Verfahren 2 nach der Ausführungsform 1 oder 2.

Ebenfalls offenbart wird eine Ausführungsform 1 einer Vorrichtung 4, erhältlich durch das Verfahren 1 nach der Ausführungsform 10 oder 11, oder durch das Verfahren 2 nach der Ausführungsform 1 oder 2.

Einen Beitrag zur Erfüllung mindestens einer der erfindungsgemäßen Aufgaben leistet eine Ausführungsform 1 eines elektrischen Geräts 1, beinhaltend ein Gehäuse, beinhaltend eine Gehäuseöffnung; wobei die Gehäuseöffnung die Vorrichtung 1 nach einer der Ausführungsformen 1 bis 19, oder die Vorrichtung 4 nach der Ausführungsform 1 umrahmt. Bevorzugt verschließt die Vorrichtung die Gehäuseöffnung hermetisch dicht.

Eine erfindungsgemäße Ausführungsform 2 des elektrischen Geräts 1 ist nach der Ausführungsform 1 ausgestaltet, wobei das elektrische Gerät ein implantierbares elektrisches medizinisches Gerät ist.

Einen Beitrag zur Erfüllung mindestens einer der erfindungsgemäßen Aufgaben leistet eine Ausführungsform 1 einer Verwendung 1 der Vorrichtung 1 nach einer der Ausführungsformen 1 bis 19, oder der Vorrichtung 4 nach der Ausführungsform 1 zu einem elektrischen Verbinden eines Inneren eines Gehäuses mit einem Äußeren des Gehäuses. Ein bevorzugtes Gehäuse ist hermetisch dicht.

Offenbart wird ferner eine Ausführungsform 1 einer Verwendung 2 des Keramikvorläufers 1 nach der Ausführungsform 1 zu einem Herstellen einer elektrischen Durchführung.

Die Offenbarung betrifft weiter eine Ausführungsform 1 eines Verfahrens 3 beinhaltend als Verfahrensschritte
a) Bereitstellen des implantierbaren elektrischen medizinischen Geräts gemäß der Ausführungsform 2 des elektrischen Geräts 1;
b) Implantieren des implantierbaren elektrischen medizinischen Geräts in einen eukaryotischen Organismus.

Ferner offenbart wird eine Ausführungsform 1 einer Verwendung 3 des implantierbaren elektrischen medizinischen Geräts nach der Ausführungsform 2 des elektrischen Geräts 1 in einer Therapie einer Krankheit oder einer Fehlfunktion oder beidem.

Bevorzugte Ausgestaltungen von Bestandteilen einer erfindungsgemäßen Kategorie, insbesondere aber nicht ausschließlich der Vorrichtungen, der Verfahren und des elektrischen Geräts, sind ebenso bevorzugt für gleichnamige oder entsprechende Bestandteile der anderen erfindungsgemäßen Kategorien.

### Cermet

Erfindungsgemäß wird als "Cermet" ein Verbundwerkstoff aus einem oder mehreren keramischen Werkstoffen in mindestens einer metallischen Matrix oder ein Verbundwerkstoff aus einem oder mehreren metallischen Werkstoffen in mindestens einer keramischen Matrix oder beides bezeichnet. Zur Herstellung eines Cermets kann beispielsweise ein Gemisch aus mindestens einem keramischen Pulver und mindestens einem metallischen Pulver verwendet werden, welches beispielsweise mindestens mit einem Vehikel, bevorzugt einem Bindemittel, und ggf. mindestens mit einem Lösungsmittel versetzt werden kann. Eine solche Mischung aus keramischen und metallischen Pulvern und Bindemittel wird auch als Cermetpaste bezeichnet. Das bzw. die keramischen Pulver des Cermets weisen vorzugsweise eine mittlere Korngröße von weniger als 10 µm, bevorzugt weniger als 5 µm, besonders bevorzugt weniger als 3 µm auf. Das bzw. die metallischen Pulver des Cermets weisen vorzugsweise eine mittlere Korngröße von weniger als 15 µm, bevorzugt weniger als 10 µm, besonders bevorzugt weniger als 5 µm auf. Als mittlere Korngröße wird dabei insbesondere der Medianwert oder D₅₀-Wert der Korngrößenverteilung angesehen. Der D₅₀-Wert beschreibt jenen Wert, bei dem 50 % der Körner des keramischen Pulvers und/oder des metallischen Pulvers feiner sind als der D₅₀-Wert. Ein bevorzugtes Cermet weist eine hohe spezifische Leitfähigkeit auf. In jeden Fall sollte das Cermet elektrisch leitfähig sein.

Die mindestens eine keramische Komponente eines erfindungsgemäßen Cermets beinhaltet bevorzugt eine erfindungsgemäße Keramik. Die mindestens eine metallische Komponente eines erfindungsgemäßen Cermets beinhaltet bevorzugt eines ausgewählt aus der Gruppe bestehend aus Platin, Iridium, Niob, Palladium, Eisen, Edelstahl, Kobald-Chrom-Legierung, Molybdän, Tantal, Wolfram, Titan, Kobalt und Zirkonium oder Kombination von mindestens zwei davon. Dabei ist eine bevorzugte Kombination eine Legierung. Ein bevorzugter Edelstahl ist ein Edelstahl 316L. Eine elektrisch leitfähige Verbindung stellt sich im Cermet in der Regel dann ein, wenn der Metallgehalt über der sogenannten Perkolationsschwelle liegt, bei der die Metallpartikel im gesinterten Cermet mindestens punktuell miteinander verbunden sind, so dass eine elektrische Leitung ermöglicht wird. Dazu sollte der Metallgehalt erfahrungsgemäß, abhängig von der Materialauswahl, mindestens 25 Vol.-% betragen, bevorzugt mindestens 32 Vol.-%, am bevorzugtesten mindestens 38 Vol.-%, jeweils bezogen auf das gesamte Volumen des Cermets.

### Cermetpaste

Eine bevorzugte Cermetpaste beinhaltet
a) eine metallische Komponente, bevorzugt mindestens ein Metallpulver, zu einem Anteil in einem Bereich von 30 bis 92 Gew.-%, bevorzugt von 40 bis 88 Gew.-%, bevorzugter von 60 bis 85 Gew.-%, jeweils bezogen auf das Gewicht der Cermetpaste,
b) eine keramische Komponente, bevorzugt mindestens ein keramisches Pulver, besonders bevorzugt mindestens ein Metalloxid, zu einem Anteil in einem Bereich von 3 bis 25 Gew.-%, bevorzugt von 4 bis 20 Gew.-%, bevorzugter von 5 bis 15 Gew.-%, jeweils bezogen auf das Gewicht der Cermetpaste, und
c) ein Vehikel als Restbestandteil bis zu 100 Gew.-%, bezogen auf das Gewicht der Cermetpaste.

### Zusammensetzungen

Eine bevorzugte erfindungsgemäße Zusammensetzung, insbesondere eine bevorzugte erste Zusammensetzung, ist eine Cermetpaste. Dies gilt insbesondere auch für alle weiteren ersten Zusammensetzungen. Eine weitere bevorzugte zweite Zusammensetzung oder weitere zweite Zusammensetzung oder beides ist eine Metallpaste. Eine bevorzugte Metallpaste beinhaltet einen Metallanteil in einem Bereich von 70 bis 95 Gew.-%, bevorzugt von 75 bis 90 Gew.-%, bevorzugter von 80 bis 90 Gew.-%, bezogen auf das Gewicht der Metallpaste, und ein Vehikel als Restbestandteil bis zu 100 Gew.-%, bezogen auf das Gewicht der Metallpaste. Es ist weiterhin bevorzugt, dass hier der Anteil an einer keramischen Komponente, vorzugsweise an einem keramischen Pulver und besonders bevorzugt an einem Metalloxid, unter 3 Gew.-%, bezogen auf das Gewicht der Metallpaste, liegt.

### Keramikvorläuferschicht

Eine bevorzugte Keramikvorläuferschicht ist eine Folie. Eine weitere bevorzugte Keramikvorläuferschicht hat eine Dicke in einem Bereich von 50 bis 2000 µm, bevorzugt von 100 bis 800 µm, bevorzugter von 200 bis 600 µm, bevorzugter von 300 bis 500 µm, am bevorzugtesten von 350 bis 450 µm. Bevorzugt werden die miteinander kontaktierten Keramikvorläuferschichten vor dem Brennen in Verfahrensschritt d) so in kleinere Einheiten zerteilt, dass ein Auftreten von thermischen Spannungen innerhalb der Keramikvorläuferschichten während des Brennens vermindert, bevorzugt minimiert, wird.

### Vehikel

Bevorzugte Vehikel sind anorganische Vehikel und organische Vehikel. Ein bevorzugtes anorganisches Vehikel ist Wasser. Ein bevorzugtes organisches Vehikel ist eine Lösung, eine Emulsion oder eine Dispersion, basierend auf einem oder mehreren Lösungsmitteln, bevorzugt einem organischen Lösungsmittel, welches gewährleistet, dass die Bestandteile der Paste in einem dispergiert oder emulgiert vorliegen. Ein weiteres bevorzugtes organisches Lösungsmittel gewährleistet eine optimale Stabilität der Pastenbestandteile und verleiht der Paste eine Viskosität, welche ein effektives Drucken der Paste ermöglicht. Ein weiteres bevorzugtes organisches Vehikel beinhaltet:
a) einen Binder, bevorzugt zu einem Anteil in einem Bereich von 1 bis 10 Gew.-%, bevorzugt von 2 bis 8 Gew.-%, bevorzugter von 3 bis 7 Gew.-%, bezogen auf das Gewicht des Vehikels,
b) ein Tensid, bevorzugt zu einem Anteil in einem Bereich von 0 bis 10 Gew.-%, bevorzugt von 0 bis 8 Gew.-%, bevorzugter von 0,01 bis 6 Gew.-%, bezogen auf das Gewicht des Vehikels,
c) ein oder mehrere Lösungsmittel, wobei der Anteil durch die Anteile der anderen Komponenten des Vehikels vorgegeben ist,
d) optional Additive, bevorzugt zu einem Anteil in einem Bereich von 0 bis 15 Gew.-%, bevorzugt von 0 bis 13 Gew.-%, bevorzugter von 5 bis 11 Gew.-%, bezogen auf das Gewicht,
wobei die Werte in Gew.-% sich zu 100 Gew.-% summieren. Ein erfindungsgemäß bevorzugtes organisches Vehikel ist eines, welches zu einer guten Druckbarkeit der Paste beiträgt.

### Binder

Bevorzugte Binder tragen zu einer geeigneten Stabilität, Druckbarkeit, Viskosität, oder einem geeigneten Sinterverhalten der Paste bei. Binder sind im Stand der Technik bekannt. Alle dem Fachmann zum erfindungsgemäßen Einsatz in einem organischen Vehikel geeignet erscheinenden Binder können verwendet werden. Erfindungsgemäß bevorzugte Binder sind Polymerbinder, Monomerbinder und Binder, die eine Kombination aus Polymeren und Monomeren sind. Polymerbinder können auch Co-Polymere sein, wobei mindestens zwei Monomereinheiten in einem einzelnen Molekül beinhaltet sind. Bevorzugte Polymerbinder tragen eine funktionelle Gruppe in ihrer Polymerhauptkette, haben eine funktionelle Gruppe außerhalb der Polymerhauptkette oder haben funktionelle Gruppen in der Polymerhauptkette und außerhalb. Bevorzugte Polymere, welche eine funktionelle Gruppe in ihrer Polymerhauptkette tragen sind zum Beispiel Polyester, substituierte Polyester, Polycarbonate, substituierte Polycarbonate, Polymere mit ringförmigen Gruppen in der Polymerhauptkette, Polyzucker, substituierte Polyzucker, Polyurethane, substituierte Polyurethane, Polyamide, substituierte Polyamide, Phenolharze, substituierte Phenolharze, Co-Polymere der Monomere von einem oder mehreren der vorgenannten Polymere, optional mit anderen Co-Monomeren, oder eine Kombination aus mindestens zwei der Vorgenannten. Bevorzugte Polymere mit ringförmigen Gruppen in der Hauptkette sind zum Beispiel Polyvinylbutylat (PVB) und seine Derivate und Polyterpineol und seine Derivate oder Mischungen der Vorgenannten. Bevorzugte Polyzucker sind beispielsweise Zellulose und Alkylderivate dessen, bevorzugt Methylzellulose, Ethylzellulose Propylzellulose, Butylzellulose, sowie deren Derivate und Mischungen aus mindestens zwei der Vorgenannten. Bevorzugte Polymere, welche eine funktionelle Gruppe außerhalb der Hauptkette tragen sind solche, die Amidgruppen tragen, die Säuregruppen und/oder Estergruppen tragen, oft Acrylharze genannt, oder Polymere, die eine Kombination der vorgenannten Gruppen tragen. Bevorzugte Polymere, welche eine Amidgruppe außerhalb der Hauptkette trage sind beispielsweise Polyvinyl-Pyrrolidon (PVP) und dessen Derivate. Bevorzugte Polymere, die eine Säuregruppe und/oder Estergruppe außerhalb der Hauptkette tragen sind beispielsweise Polyacrylsäure sowie deren Derivate, Polymethacrylsäure (PMA) und deren Derivate oder Polymethylmethacrylat (PMMA) und dessen Derivate, oder Mischungen aus den Vorgenannten. Erfindungsgemäß bevorzugte monomerische Binder sind ethylenglycolbasierte Monomere, Terpineolharze sowie Rosinderivate oder Mischungen der Vorgenannten. Bevorzugte ethylenglycolbasierte Monomere sind solche mit Ethergruppen, Estergruppen oder solche mit Ethergruppen und Estergruppen, bevorzugte Ethergruppen sind hierbei Methyl, Ethyl, Propyl, Butyl, Pentyl, Hexyl und höhere Alkyle, bevorzugte Estergruppen sind Acetate und deren Alkylderivate, bevorzugt Ethylenglucol-Monobutylether-Monoacetate oder Mischungen der Vorgenannten. Besonders bevorzugte Binder sind Ethylzellulose und deren Derivate sowie Mischungen mit weiteren Bindern aus den oben aufgeführten sowie anderen.

### Tensid

Bevorzugte Tenside tragen zu einer geeigneten Stabilität, Druckbarkeit, Viskosität, oder einem geeigneten Sinterverhalten der Paste bei. Tenside sind im Stand der Technik bekannt. Alle dem Fachmann zum erfindungsgemäßen Einsatz in einem organischen Vehikel geeignet erscheinenden Tenside können verwendet werden. Bevorzugte Tenside basieren auf linearen Ketten, verzweigten Ketten, aromatischen Ketten, fluoridierten Ketten, Siloxanketten, Polyetherketten, oder Kombinationen aus mindestens zwei davon. Bevorzugte Tenisde sind einfachkettig, doppelkettig, oder polykettig. Bevorzugte Tenside haben nichtionische, anionische, kationische, oder zwitterionische Enden. Weitere bevorzugte Tenside sind polymerisch oder monomerisch oder eine Mischung aus beidem. Bevorzugte Tenside können pigmentaffine Gruppen haben, bevorzugt hydroxyfunktionale Carbonsäureester mit pigmentaffinen Gruppen (zum Beispiel DISPERBYK®-108, hergestellt von BYK USA, Inc.), Acrylatco-polymere mit pigmentaffinen Gruppen (zum Beispiel DISPERBYK®-116, hergestellt von BYK USA, Inc.), modifizierte Polyether mit pigmentaffinen Gruppen (zum Beispiel TEGO® DISPERS 655, hergestellt von Evonik Tego Chemie GmbH), und andere Tenside mit Gruppen hoher Pigmentaffinität (zum Beispiel TEGO® DISPERS 662 C, hergestellt von Evonik Tego Chemie GmbH). Andere bevorzugte Polymere sind Polyethylenglycol und seine Derivate, und Alkylcarbonsäure und ihre Derivate oder Salze, oder Mischungen aus mindestens zwei davon. Ein bevorzugtes Polyethylenglycol ist Poly(ethylenglycol-)essigsäure. Bevorzugte Alkylcarbonsäuren sind solche mit völlig gesättigten oder einfach oder mehrfach ungesättigten Alkylketten oder Mischungen aus mindestens zwei davon. Bevorzugte Carbonsäuren mit gesättigten Alkylketten sind solche mit Alkylkettenlängen in einem Bereich von 8 bis 20 Kohlenstoffatomen, bevorzugt C₉H₁₉COOH (Caprinsäure), C₁₁H₂₃COOH (Laurinsäure), C₁₃H₂₇COOH (Myristinsäure), C₁₅H₃₁COOH (Palmitinsäure), C₁₇H₃₅COOH (Stearinsäure), oder Mischungen aus mindestens zwei davon. Bevorzugte Carbonsäuren mit ungesättigten Alkylketten sind C₁₈H₃₄O₂ (Ölsäure) und C₁₈H₃₂O₂ (Linolsäure).

### Lösungsmittel

Ein erfindungsgemäß bevorzugtes Lösungsmittel ist eine Komponente der Paste, welche während des Brennens im Wesentlichen aus der Paste entfernt wird. Bevorzugt wird dere Anteil des Lösungsmittel an der Paste während des Brennens um mindestens 80 Gew.-%, bevorzugt um mindestens 95 Gew.-%, bezogen auf das Gewicht der Paste bzw. des gebrannten Bestandteils gegenüber vor dem Brennen reduziert. Bevorzugte Lösungsmittel tragen zu einer geeigneten Stabilität, Druckbarkeit, Viskosität, oder einem geeigneten Sinterverhalten der Paste bei. Lösungsmittel sind im Stand der Technik bekannt. Alle dem Fachmann zum erfindungsgemäßen Einsatz in einem organischen Vehikel geeignet erscheinenden Lösungsmittel können verwendet werden. Ein bevorzugtes Lösungsmittel liegt bei Raumtemperatur und Normaldruck(298.15 K, 100 kPa) als Flüssigkeit vor. Ein weiteres bevorzugtes Lösungsmittel hat eine Siedetemperatur über 90°C und eine Schmelztemperatur über -20°C. Ein weiteres bevorzugtes Lösungsmittel ist eine polare oder unpolare, protische oder aprotische, aromatische oder nicht aromatische oder ionische Flüssigkeit. Bevorzugte Lösungsmittel sind Monoalkohole, Dialkohole, Polyalkohole, Monoester, Diester, Polyester, Monoether, Diether, Polyether, Lösungsmittel beinhaltend eine oder mindestens zwei funktionelle Gruppen dieser Kategorien, optional beinhaltend funktionelle Gruppen anderer Kategorien, bevorzugt cyclische Gruppen, aromatische Gruppen. ungesättigte Bindungen, Alkoholgruppen in denen ein oder mindestens zwei Sauerstoffatome ersetzt sind durch Heteroatome, Ethergruppen in denen ein oder mindestens zwei Sauerstoffatome ersetzt sind durch Heteroatome, Estergruppen in denen ein oder mindestens zwei Sauerstoffatome ersetzt sind durch Heteroatome, und Mischungen aus mindestens zwei davon. Dabei sind bevorzugte Ester Dialkylester von Adipinsäure, mit bevorzugten Alkylbestandteilen Methyl-, Ethyl-, Propyl-, Butyl-, Pentyl-, Hexyl-, und höhere Alkylgruppen oder Mischungen aus mindestens zwei davon, bevorzugt Dimethyladipin, und Mischungen aus zwei oder mehr Adipinestern. Bevorzugte Ether sind Diether, bevorzugt Dialkylether von Ethylenglycol, mit bevorzugten Alkylbestandteilen Methyl-, Ethyl-, Propyl-, Butyl-, Pentyl-, Hexyl-, und höheren Alkylgruppen oder Mischungen aus mindestens zwei davon, und Mischungen aus zwei Diethern. Bevorzugte Alkohole sind primäre, sekundäre und tertiäre Alkohole, oder eine Mischung aus zwei oder mehr Alkoholen. Ein bevorzugter Alkohol ist Terpineol und seine Derivate. Bevorzugte Alkohole, welche zwei oder mehr funktionelle Gruppen beinhalten sind 2,2,4-Trimethyl-1,3-Pentandiol-Mono-Iso-Butyrat, auch Texanol genannt, und dessen Derivate, 2-(2-Ethoxyethoxy)Ethanol, auch Carbitol genannt, dessen Alkylderivate, bevorzugt Methyl-, Ethyl-, Propyl-, Butyl-, Pentyl-, Hexylcarbitol, bevorzugt Hexylcarbitol oder Butylcarbitol, und Acetatderivate dessen, bevorzugt Butylcarbitolacetat, oder Mischungen aus mindestens zwei der Vorgenannten.

### Additive im organischen Vehikel

Bevorzugte Additive im organischen Vehikel sind von den vorgenannten Komponennten des Vehikels verschieden. Bevorzugte Additive tragen zu einer geeigneten Stabilität, Druckbarkeit, Viskosität, oder einem geeigneten Sinterverhalten der Paste bei. Vehikeladditive sind im Stand der Technik bekannt. Alle dem Fachmann zum erfindungsgemäßen Einsatz in einem organischen Vehikel geeignet erscheinenden Additive können verwendet werden. Erfindungsgemäß bevorzugte Additive sind Thixotropiermittel, Viskositätsregulatoren, Stabilisierungsmittel, anorganische Additive, Verdickungsmittel, Emulgiermittel, Dispergiermittel, und pHregulatoren. Bevorzugte Thixotropiermittel sind Carbonsäurederivate, bevorzugt Fettsäurederivate. Bevorzugte Fettsäurederivate sind C₉H₁₉COOH (Caprinsäure), C₁₁H₂₃COOH (Laurinsäure), C₁₃H₂₇COOH (Myristinsäure), C₁₅H₃₁COOH (Palmitinsäure), C₁₇H₃₅COOH (Stearinsäure), C₁₈H₃₄O₂ (Ölsäure) und C₁₈H₃₂O₂ (Linolsäure), oder Mischungen aus mindestens zwei davon. Eine bevorzugte Mischung, beinhaltend Fettsäurederivate, ist Castoröl.

### Metall

Hier kommen alle dem Fachmann geläufigen Metalle in Betracht, die neben einer Leitfähigkeit auch eine gute Verträglichkeit mit eukaryotischem Gewebe aufweisen. Ein erfindungsgemäß bevorzugtes Metall ist vorzugsweise ausgewählt aus der Gruppe bestehend aus Platin, Iridium, Niob, Palladium, Eisen, Edelstahl, Kobald-Chrom-Legierung, Molybdän, Tantal, Wolfram, Titan, Kobalt und Zirkonium oder eine Kombination von mindestens zwei davon. Dabei ist eine bevorzugte Kombination eine Legierung. Ein bevorzugter Edelstahl ist ein Edelstahl 316L. Ein bevorzugtes Metall ist biokompatibel. Eine bevorzugte Legierung ist biokompatibel.

### Keramik

Eine erfindungsgemäße Keramik kann jede Keramik sein, die der Fachmann für den erfindungsgemäßen Einsatz auswählen würde. Die Keramik ist bevorzugt ausgewählt aus der Gruppe bestehend aus einer Oxidkeramik, einer Silikatkeramik, einer Nichtoxid-Keramik oder einer Mischung aus mindestens zwei davon.

Die Oxidkeramik ist bevorzugt ausgewählt aus der Gruppe bestehend aus einem Metalloxid, einem Halbmetalloxid oder einer Mischung davon. Das Metall des Metalloxids kann ausgewählt sein aus der Gruppe bestehend aus Aluminium, Beryllium, Barium, Calcium, Magnesium, Natrium, Kalium, Eisen, Zirkonium, Titan oder einer Mischung von mindestens zwei davon. Das Metalloxid ist bevorzugt ausgewählt aus der Gruppe bestehend aus Aluminiumoxid (Al₂O₃), Magnesiumoxid (MgO), Zirkoniumoxid (ZrO₂), Yttriumoxid (Y₂O₃), Aluminiumtitanat (Al₂TiO₅), einer Piezokeramik wie Blei-Zirkonat (PbZrO₃), Blei-Titanat (PbTiO₃) sowie Blei-Zirkonat-Titanat (PZT) oder einer Mischung von mindestens zwei hiervon. Das Halbmetall des Halbmetalloxids ist bevorzugt ausgewählt aus der Gruppe bestehend aus Bor, Silicium, Arsen, Tellur oder einer Mischung von mindestens zwei davon. Eine weitere bevorzugte Oxidkeramik beinhaltet eines ausgewählt aus der Gruppe bestehend aus Zirkoniumoxid verstärktes Aluminiumoxid (ZTA - Zirconia Toughened Aluminum - Al₂O₃/ZrO₂), Yttrium verstärktes Zirkoniumoxid (Y-TZP), Barium(Zr, Ti)oxid, Barium(Ce, Ti)oxid oder eine Kombination aus mindestens zwei davon.

Die Silikatkeramik ist bevorzugt ausgewählt aus der Gruppe bestehend aus einem Steatit (Mg₃[Si₄O₁₀(OH)₂]), Cordierit (Mg, Fe²⁺)₂(Al₂Si)[Al₂Si₄O₁₈]), Mullit (Al₂Al₂₊₂ₓSi₂₋₂ₓO₁₀₋ₓ mit x = Sauerstoffleerstellen pro Elementarzelle), Feldspat (Ba,Ca,Na,K,NH₄)(Al,B,Si)₄O₈) oder einer Mischung aus mindestens zwei davon.

Die Nichtoxid-Keramik kann ausgewählt sein aus der Gruppe bestehend aus einem Carbid, einem Nitrid oder einer Mischung daraus. Das Carbid kann ausgewählt sein aus der Gruppe bestehend aus Siliciumcarbid (SiC), Borcarbid (B₄C), Titancarbid (TiC), Wolframcarbid, Zementit (Fe3C). Das Nitrid kann ausgewählt sein aus der Gruppe bestehend aus Siliciumnitrid (Si₃N₄), Aluminiumnitrid (AlN), Titannitrid (TiN), Siliciumaluminiumoxinitrid (SIALON) oder einer Mischung aus mindestens zwei davon. Eine weitere bevorzugte Nichtoxid-Keramik ist Natrium-Kalium-Niobat.

### biokompatibles Material

Ein bevorzugtes biokompatibles Material ist eines ausgewählt aus der Gruppe bestehend aus biotolerant, bioinert und bioaktiv oder eine Kombination aus mindestens zwei davon.

### Bestandteile

Bevorzugt ist die Tunnelfüllung, welche die Bestandteile beinhaltet, einstückig ausgebildet. Bevorzugt sind dabei die Bestandteile gemeinsam zu einem Stück gebrannt worden. Bevorzugt bilden die Bestandteile einer Tunnelfüllung einen Schichtaufbau, in dem die Bestandteile als einander überlagernde Schichten vorliegen. Ein bevorzugter erster Bestandteil hat eine erste elektrische Leitfähigkeit in einem Bereich von 1·10³ bis 1·10⁶ S/m, bevorzugt von 10·10³ bis 5·10⁵ S/m, bevorzugter von 5·10⁴ bis 5·10⁵ S/m. Ein bevorzugter zweiter Bestandteil oder eine bevorzugter dritter Bestandteil oder bevorzugt beide haben eine zweite bzw. dritte elektrische Leitfähigkeit in einem Bereich von 1·10³ bis 1·10⁷ S/m, bevorzugt von 10·10³ bis 1·10⁷ S/m, bevorzugter von 1·10⁵ bis 1·10⁷ S/m. Ein bevorzugter Keramikkörper hat eine weitere elektrische Leitfähigkeit in einem Bereich von 1·10⁻⁶ bis 1·10⁻⁵ S/m, bevorzugt von 10·10⁻⁶ bis 50·10⁻⁴ S/m, bevorzugter von 18·10⁻⁶ bis 18·10⁻⁴ S/m.

### hermetisch dicht

Ein Tunnel der erfindungsgemäßen Vorrichtung ist bevorzugt hermetisch dicht verschlossen. Eine erfindungsgemäße Vorrichtung oder ein erfindungsgemäßes elektrisches Gerät ist bevorzugt hermetisch dicht. Im Rahmen der Erfindung kann der Begriff "hermetisch dicht" dabei verdeutlichen, dass bei einem bestimmungsgemäßen Gebrauch innerhalb üblicher Zeiträume (beispielsweise 5 bis 10 Jahre) Feuchtigkeit oder Gase oder beides nicht oder nur minimal durch den hermetisch dichten Körper hindurch zwischen innen und außen ausgetauscht werden können. Eine physikalische Größe, die beispielsweise eine Permeation von Gasen oder Feuchtigkeit oder beidem durch den Körper beschreiben kann, ist die sogenannte Leckrate, welche beispielsweise durch Lecktests bestimmt werden kann. Entsprechende Lecktests können beispielsweise mit Heliumlecktestern und/oder Massenspektrometern durchgeführt werden und sind im Standard Mil-STD-883G Method 1014 spezifiziert. Die maximal zulässige Helium-Leckrate wird dabei abhängig vom internen Volumen des zu prüfenden Körpers, hier zum beispiel dem Innenvolumen des elektrischen Geräts, festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, können diese maximal zulässigen Helium-Leckraten beispielsweise von 1 x 10⁻⁸ atm×cm³/s bis 1 x 10⁻⁷ atm×cm³/s betragen. Im Rahmen der Erfindung kann der Begriff "hermetisch dicht" insbesondere bedeuten, dass das elektrische Gerät oder die erfindungsgemäße Vorrichtung in einem ansonsten perfekt hermetisch dichten Gehäuse eine Helium-Leckrate von weniger als 1 x 10⁻⁷ atm×cm³/s aufweist. In einer vorteilhaften Ausführung kann die Helium-Leckrate weniger als 1 x 10⁻⁸ atm×cm³/s, insbesondere weniger als 1 x 10⁻⁹ atmcm³/s betragen.

Zum Zweck der Standardisierung können die genannten Helium-Leckraten auch in die äquivalente Standard-Luft-Leckrate konvertiert werden. Die Definition für die äquivalente Standard-Luft-Leckrate (Equivalent Standard Air Leak Rate) und die Umrechnung sind im Standard ISO 3530 angegeben. Aufgrund der Einsatzart von implantierbaren Therapiegeräten ist deren hermetische Dichtigkeit und Biokompatibilität in der Regel eine der vorrangigsten Anforderungen. Das hier vorgeschlagene elektrische Gerät kann insbesondere in einen Körper eines menschlichen oder tierischen Benutzers, insbesondere eines Patienten, eingesetzt werden. Dadurch ist das Gerät in der Regel einer Flüssigkeit eines Körpergewebes des Körpers ausgesetzt. Somit ist es in der Regel von Bedeutung, dass weder Körperflüssigkeit in das Gerät eindringt, noch das Flüssigkeiten aus dem Gerät austreten. Um dieses sicherzustellen, sollte das Gerät eine möglichst vollständige Undurchlässigkeit aufweisen, insbesondere gegenüber Körperflüssigkeiten.

### isostatisches Pressen

Ein isostatisches Pressen ist ein Pressen eines Werkstücks, wobei ein Unterschied der Drücke, die aus jeder Raumrichtung auf das Werkstück ausgeübt werden, möglichst gering ist. Ein bevorzugtes isostatisches Pressen erfolgt bei einer Temperatur des zu pressenden Werkstücks, bevorzugt der Keramikvorläuferschichten, in einem Bereich von 50 bis 100°, bevorzugt von 60 bis 90°, bevorzugter von 65 bis 75°C. Bevorzugt befindet sich ein zu pressendes Werkstück, bevorzugt die Keramikvorläuferschichten, bei einem isostatischen Pressen in einer Flüssigkeit, bevorzugt einem Ölbad.

### Kontaktieren / Laminieren

Ein bevorzugtes Kontaktieren ist ein Laminieren. Durch ein Laminieren werden mindestens zwei Schichten oder zwei Oberflächen fest miteinander verbunden. Bevorzugt erfolgt das Laminieren durch Verpressen. Es wird also ein Laminat erzeugt, wobei ein Laminat ein flächenförmiger Verbund ist. Ein bevorzugtes Laminieren beinhaltet ein isostatisches Pressen. Zwei Gegenstände sind hierbei fest miteinander fest verbunden, wenn ihre starre Haftung aneinander über Van-der-Waals-Kräfte hinausgeht.

### Einbringen / Überlagern

Ein bevorzugtes Einbringen einer Zusammensetzung oder ein bevorzugtes Überlagern mit einer Zusammensetzung in dem erfindungsgemäßen Verfahren erfolgt als ein Rakeln oder ein Drucken oder beides der Zusammensetzung. Ein bevorzugtes Drucken ist ein Siebdrucken oder ein Schablonendrucken oder beides.

### Brennen

Das erfindungsgemäße Brennen kann in jedem dem Fachmann zum Brennen eines Grünlings geeignet erscheinenden Ofen durchgeführt werden. Ein bevorzugtes Brennen erfolgt in einem Kammerofen. Ein weiteres bevorzugtes Brennen beinhaltet eine Maximaltemperatur in einem Bereich von 1000 bis 2000°C, bevorzugt von 1250 bis 1900°C, bevorzugter von 1510 bis 1650°C. Ein weiteres bevorzugtes Brennen beinhaltet eine Haltedauer eines Haltens einer Maximaltemperatur in einem Bereich von 0,3 bis 10 h, bevorzugt von 0,5 bis 7 h, bevorzugter von 1 bis 5 h. Ein weiteres bevorzugtes Brennen ist ein Sintern

### Sintern

Unter einem Sintern oder einem Sinterprozess wird im Rahmen der vorliegenden Erfindung allgemein ein Verfahren zur Herstellung von Werkstoffen oder Werkstücken verstanden, bei welchem pulverförmige, insbesondere eines ausgewählt aus der Gruppe bestehend aus feinkörnige Stoffe, keramische Stoffe und metallische Stoffe oder eine Kombination aus mindestens zwei davon erhitzt werden und dadurch verbunden werden. Dieser Prozess kann ohne äußeren Druck auf den zu erhitzenden Stoff erfolgen oder kann insbesondere unter erhöhtem Druck auf den zu erhitzenden Stoff erfolgen, beispielsweise unter einem Druck von mindestens 2 bar, vorzugsweise höheren Drucken, beispielsweise Drucken von mindestens 10 bar, insbesondere mindestens 100 bar oder sogar mindestens 1000 bar. Der Prozess kann insbesondere vollständig oder teilweise bei Temperaturen unterhalb der Schmelztemperatur der pulverförmigen Werkstoffe erfolgen, beispielsweise bei Temperaturen von 700°C bis 1400 °C. Der Prozess kann insbesondere vollständig oder teilweise in einem Werkzeug oder einer Form oder beides durchgeführt werden, so dass mit dem Sinterprozesses eine Formgebung verbunden werden kann. Neben den pulverförmigen Werkstoffen kann ein Ausgangsmaterial für den Sinterprozess weitere Werkstoffe umfassen, beispielsweise einen oder mehrere Binder, oder ein oder mehrere Lösungsmittel, oder beides. Der Sinterprozess kann in einem Schritt oder auch in mehreren Schritten erfolgen, wobei dem Sinterprozess beispielsweise weitere Schritte vorgelagert sein können, beispielsweise ein oder mehrere Formgebungsschritte, oder ein oder mehrere Entbinderungsschritte, oder beides. Das Sintern bzw. der Sinterprozess entspricht somit einem Brennprozess. Der Sinterprozess, insbesondere für ein Cermet, kann vergleichbar zu einem üblicherweise für homogene Pulver verwendeten Sinterprozess ablaufen. Beispielsweise kann unter hoher Temperatur und ggf. hohem Druck das Material beim Sintervorgang verdichtet werden, so dass das Cermet nahezu dicht ist, oder eine höchstens geschlossene Porosität aufweist. Cermets zeichnen sich in der Regel durch eine besonders hohe Härte und Verschleißfestigkeit aus.

### Trocknen

Ein bevorzugtes Trocknen beinhaltet eine Maximaltemperatur in einem Bereich von 30 bis 500°C, bevorzugt von 40 bis 400°C, bevorzugter von 45 bis 300°C, bevorzugter von 50 bis 200°C, bevorzugter von 55 bis 100°C, bevorzugter von 60 bis 95°C, bevorzugter von 70 bis 90°C, am bevorzugtesten von 75 bis 85°C. Ein weiteres bevorzugtes Trocknen wird für mindestens 3 min, bevorzugt mindestens 4 min, bevorzugter mindestens 5 min, bevorzugter mindestens 7 min, am bevorzugtesten mindestens 10 min, durchgeführt.

### implantierbares elektrisches medizinisches Gerät

Ein bevorzugtes implantierbares elektrisches medizinisches Gerät ist eine Therapiegerät oder eine Diagnosegerät oder beides. Ein bevorzugtes Therapiegerät ist in ein Herz implantierbar. Ein weiteres bevorzugtes Therapiegerät ist ein Defibrillator oder ein Schrittmacher oder beides. Ein bevorzugter Schrittmacher ist einer ausgewählt aus der Gruppe bestehend aus einem Herzschrittmacher, einem Blasenschrittmacher, einem Darmschrittmacher, einem Hirnschrittmacher, einem Atemschrittmacher und einem Zwerchfell schrittmacher oder eine Kombination aus mindestens zwei davon. Ein besonders bevorzugter Schrittmacher ist ein Herzschrittmacher. Ein bevorzugter Herzschrittmacher ist ein kabelloser Herzschrittmacher. Ein bevorzugtes Diagnosegerät ist ein Biomonitor. Ein bevorzugter Biomonitor beinhaltet eines ausgewählt aus der Gruppe bestehend aus einem EKG-Gerät, einem Holter-Monitor, einem Event-Recorder, und einem Loop-Recorder, oder eine Kombination von mindestens zwei davon. Ein bevorzugtes EKG-Gerät ist ein Langzeit-EKG-Gerät, welches bevorzugt Daten speichert, die über einen Zeitraum von mindestens einer Stunde anfallen. Ein weiteres bevorzugtes Diagnosegerät beinhaltet eine Sendeeinrichtung oder einen Datenspeicher oder beides. Eine bevorzugte Sendeeinrichtung ist ausgebildet zu einem drahtlosen, bevorzugt telemetrischen, Übertragen von Daten, bevorzugt EKG-Daten. Ein bevorzugtes drahtloses Übertragen von Daten ist ein Übertragen mittels Wellen. Bevorzugte Wellen sind Longitudinalwellen oder Transversalwellen oder beides. Bevorzugte Longitudinalwellen sind akustische Wellen oder Schallwellen oder beides. Bevorzugte Transversalwellen sind elektromagnetische Wellen. Bevorzugte elektromagnetische Wellen sind Wellen der Frequenz eines Mobilfunknetzes oder von Bluetooth oder beides. Ein bevorzugtes Mobilfunknetz ist ein GSM-Netz. Als Datenspeicher kann jede Einheit zum Speichern von Daten ausgewählt werden, welche der Fachmann für geeignet zum Speichern medizinischer Daten, bevorzugt EKG-Daten, in einem implantierbaren Gerät hält. Ein bevorzugter Datenspeicher ist ein Magnetspeicher oder ein Flashspeicher oder beides. Ein weiteres bevorzugtes implantierbares elektrisches medizinisches Gerät beinhaltet einen elektrischen Impulsgenerator.

### elektrischer Impulsgenerator

Ein elektrischer Impulsgenerator ist eine elektronische Schaltung oder ein elektronisches Gerät oder beides, welche oder welches ausgebildet ist um einmalig oder wiederholt für einen kurzen Zeitraum einen elektrischen Spannungspuls abzugeben. Ein bevorzugter elektrischer Spannungspuls ist ein Gleichspannungspuls. Ein weiterer bevorzugter Spannungspuls hat einen maximalen Spannungswert von weniger als 24 V, bevorzugt weniger als 12 V, bevorzugter weniger als 10 V, am bevorzugtesten weniger als 2,4 V. Ein bevorzugter kurzer Zeitraum ist kürzer als 500 ms, bevorzugt kürzer als 100 ms, bevorzugter kürzer als 50 ms, am bevorzugtesten kürzer als 10 ms.

### eukaryotisches Gewebe

Ein bevorzugtes eukaryotisches Gewebe ist ein tierisches Gewebe oder ein menschliches Gewebe oder beides.

### Therapie / Krankheit / Fehlfunktion

Eine bevorzugte Therapie ist eine Neurotherapie oder eine Herztherapie oder beides. Hierbei beinhaltet eine Neurotherapie bevorzugt eine elektrische Stimulation eines Nervs. Eine bevorzugte Herztherapie beinhaltet bevorzugt eine elektrische Stimulation eines Herzens. Eine bevorzugte Neurotherapie ist eine ausgewählt aus der Gruppe bestehend aus einer tiefen Hirnstimulation (Deep Brain Stimulation - DBS), einer Rückenmarksstimulation, einer Vagusnervstimulation, einer Sacralnervstimulation und einer Magen -oder Darmnervenstimulation oder eine Kombination aus mindestens zwei davon. Eine bevorzugte Herztherapie ist eine ausgewählt aus der Gruppe bestehend aus einer Herzschrittmachertherapie, einer ICD-Therapie, einer CRT-P-Therapie und einer CRT-D-Therapie oder eine Kombination aus mindestens zwei davon. Eine bevorzugte tiefe Hirnstimulation ist eine Therapie von einem ausgewählt aus der Gruppe bestehend aus Alzheimer, einer Parkinson-Krankheit, einem Tremor, einer Depression, einer Epilepsie, einer Dystonie, einer Zwangsstörung, einem Tourettesyndrom, einem Koma und einem Trauma oder eine Kombination aus mindestens zwei davon. Eine bevorzugte Rückenmarksstimulation ist eine Therapie von einem ausgewählt aus der Gruppe bestehend aus chronischem Rückenschmerzen, einem Postdiskotomiesyndrom, einem komplexen regionalen Schmerzsyndrom und einem therapieresistenten Schmerz, bevorzugt durch Ischämie, oder eine Kombination aus mindestens zwei davon. Eine bevorzugte Vagusnervstimulation ist eine Therapie von einem ausgewählt aus der Gruppe bestehend aus einer Epilepsie, einer Depression und einem chronischen Herzfehler (Chronic Heart Failure - CHF) oder eine Kombination aus mindestens zwei davon. Eine bevorzugte Sacralnervstimulation ist eine Therapie von einem ausgewählt aus der Gruppe bestehend aus einer Harninkontinenz, einem Harnverhalt, einem häufigen Harndrang, einer Stuhlinkontinenz, einer idiopathischen Obstipation, einer interstitiellen Cystitis und einer chronischen Analfissur oder eine Kombination aus mindestens zwei davon. Eine bevorzugte Magen -oder Darmnervenstimulation ist eine Therapie von Fettleibigkeit. Eine bevorzugte Herzschrittmachertherapie ist eine Therapie von Bradykardie oder Tachykardie oder beidem. Eine bevorzugte ICD-Therapie ist eine Therapie von Kammerflimmern oder ventrikulärer Tachykardie oder beidem. Eine bevorzugte CRT-P-Therapie ist eine Therapie eines chronischen Herzfehlers. Eine bevorzugte CRT-D-Therapie ist eine Therapie einer herzfehlerinduzierten Leitungsstörung oder einer ventrikulären Dyssynchronie oder beidem.

### MESSMETHODEN

Die folgenden Messmethoden wurden im Rahmen der Erfindung benutzt. Sofern nichts anderes angegeben ist wurden die Messungen bei einer Umgebungstemperatur von 25°C, einem Umgebungsluftdruck von 100 kPa (0,986 atm) und einer relativen Luftfeuchtigkeit von 50 % durchgeführt.

### Biokompatibilität

Die Biokompatibilität wird gemäß der Norm nach 10993-4:2002 bestimmt.

### elektrische Leitfähigkeit

Die elektrische Leitfähigkeit wird mit einem handelsüblichen Leitfähigkeitsmessgerät (GLF 100 Universal-Leitfähigkeitsmessgerät von GHM Messtechnik GmbH Standort Greisinger, Regenstauf, Deutschland) gemessen.

### Viskosität

Die Viskosität wurde mit einem Thermo Fischer Scientific Corp. "Haake Rheostress 6000", ausgestattet mit einer Grundplatte MPC60 Ti und einem Kegel C 20/0,5° Ti sowie der Software "Haake RheoWin Job Manager 4.00.0003". Zunächst wurde der Abstandsnullpunkt eingestellt. Dann wurde eine für die Messung ausreichende Probenmenge der zu vermessenden Paste auf die Grundplatte gegeben. Der Kegel wurde in die Messposition mit einem Abstandspalt von 0,026 mm geführt und überschüssiges Material mit einem Spatel entfernt. Die Probe wurde für 3 Minuten auf 25°C temperiert und die Rotationsmessung gestartet. Die Scherrate wurde innerhalb von 48 s in 24 äquidistanten Messschritten von 0 auf 20 s⁻¹ erhöht und dann innerhalb von 312 s in 156 äquidistanten Messschritten weiter auf 150 s⁻¹ erhöht. Nach einer Wartezeit von 60 s bei einer Scherrate von 150 s⁻¹ wurde die Scherrate innerhalb von 312 s und 156 äquidistanten Messschritten auf 20 s⁻¹ reduziert und dann innerhalb von 48 s in 24 äquidistanten Messschritten weiter auf 0 s⁻¹ abgesenkt. Die Drehmomentenkorrektur, Druckkontrolle und Massenträgheitskorrektur waren bei der Messung aktiviert. Die Viskosität ist durch den Messwert bei der Scherrate von 100 s⁻¹, welcher während des Absenkens der Scherrate erreicht wurde, gegeben.

### Rastereleketronenmikroskopie (scanning electron microscopy - SEM)

Durch die Probe wurde ein Schnitt erzeugt, so dass die zu untersuchende Fläche frei gelegt wurde. Im Allgemeinen erfolgte der Schnitt durch die Schichten des Schichtaufbaus sowie als Längsschnitt durch einen Tunnel der Probe. Somit wurde ein Querschnitt durch den Schichtaufbau und gleichzeitig ein Längsschnitt durch den Tunnel erzeugt. Die geschnittene Probe wurde in einen mit einem Einbettungsmaterial gefüllten Behälter gegeben und dabei so angeordnet, dass die zu untersuchende Fläche nach oben wies. Als Einbettungsmaterial wurde EpoFix (Struers GmbH) verwendet und gemäß der Verwendungsanleitung angemischt. Nach 8 Stunden Härten bei Raumtemperatur wurde die Probe weiterverarbeitet. In einem ersten Schritt wurde die Probe abgeschliffen mit einem Labopol-25 (Struers GmbH) sowie Siliziumkarbidpapier 180-800 (Struers GmbH) bei 250 Umdrehungen / min. In einem weiteren Schritt wurde die Probe mit einem Rotopol-2, ausgestattet mit einem Retroforce-4, MD Piano 220 und MD allegro, poliert. Die Probe wurde untersucht mit einem Zeiss Ultra 55 (Carl Zeiss AG), ausgestattet mit einer Feldemissionselektrode bei einer Beschleunigungsspannung von 20 kV und einem Druck von etwa 3×10⁻⁶ mbar. In einigen Fällen wurden die Schnitte verwendet, um die elementare Zusammensetzung entlang einer Geraden durch die verschiedenen Schichten zu bestimmen. Eine so genannte Linienanalyse (line scan) wurde mit einer EDX-Messung (energy dispersive X-ray spectroscopy) durchgeführt. Hierzu wurde ein IncaPentaFETx3 angebracht an dem Zeiss Ultra 55 sowie die Software "The Microanalysis Suite Issue 18d + SP3" (beides von Oxford Instruments) mit einer Apertur von 30 µm verwendet.

Die Erfindung wird im Folgenden durch Beispiele und Zeichnungen genauer dargestellt, wobei die Beispiele und Zeichnungen keine Einschränkung der Erfindung bedeuten. Ferner sind die schematischen Darstellungen nicht maßstabsgetreu.

### Pastenrezepturen

### Cermetpaste:

60 g eines Platinpulvers wurden mit 24 g eines Al₂O₃-Pulvers wurden mit einem organischen Vehikel auf Ethylzellulosebasis gemischt und mit einer 3-Walzenmühle homogenisiert. So erhaltene Pasten hatten Viskositäten in einem Bereich von 250 bis 300 Pa·s und eine Mahlfeinheit (fineness of grind - FoG) von weniger als 10 µm. Die Rheologie der Pasten war geeignet für den Schablonendruck.

### Metallpaste:

100 g eines Platinpulvers wurden mit 10 g eines Al₂O₃-Pulvers wurden mit einem organischen Vehikel auf Ethylzellulosebasis gemischt und mit einer 3-Walzenmühle homogenisiert. So erhaltene Pasten hatten Viskositäten in einem Bereich von 250 bis 300 Pa·s und eine Mahlfeinheit (fineness of grind - FoG) von weniger als 10 µm. Die Rheologie der Pasten war geeignet für den Schablonendruck.

### Vorbereitung der Pasten

Im Detail wurden die Pasten folgendermaßen vorbereitet. Die oben angegeben Komponenten wurden manuell in einem Glaskolben gemischt. Diese vorgemischten Pasten wurden mit einer 3-Walzenmühle Exakt E80 (von EXAKT Advanced Technologies GmbH) mit Edelstahlwalzen homogenisiert. Dazu wurden die Pasten mehrere Male durch die Mühle geführt, wobei die Spalte zwischen den Walzen (Spalt 1 zwischen der ersten und der zweiten Walze; Spalt 2 zwischen der zweiten und der dritten Walze) in ihrer Breite schrittweise verringert wurden. Die Spalte zwischen den Walzen konnten bezüglich ihrer Breite (distance control) oder dem Druck zwischen den Walzen (pressure control) reguliert werden. Die Anfangsschritte des Mahlens wurden mit distance control durchgeführt. Die Endschritte des Mahlens wurden mit pressure control durchgeführt. Die untenstehenden Werte wurden für die Spalte bei den angegeben Mahlschritten verwendet. Hierbei wurden für den Spalt 1 und den Spalt 2 jeweils gleiche Werte verwendet.
Schritte 1 und 2:: distance control 20 µm
Schritte 3 und 4: distance control 15 µm
Schritte 5 und 6: distance control 10 µm
Schritte 7 und 8: distance control 5 µm
Schritte 9, 10 und 11: pressure control 10 N/mm
Schritte 12, 13 und 14: pressure control 12.5 N/mm
Schritte 15, 16 und 17: pressure control 15 N/mm

### Vorbereitung keramischer Grünkörperfolien

Keramische Grünkörperfolien wurden als Keramikvorläufer eingesetzt. Hierzu wurden 99,7 %- reine Al₂O₃ - Folien (Keral 99 von Keramische Folien GmbH) der Dicke 400 µm verwendet. Proben der Grünkörperfolien wurden zu 90 mm × 90 mm-Quadraten zugeschnitten. Etwa kreisrunde Löcher mit einem Durchmesser von 400 µm wurden mit einem mechanischen Stanzeisen (CPC923101 von Groz-Beckert KG) für 400 µm Durchmesser in einer automatischen Stanze (MP4150 Stanze von Unichem Industries Inc.) in die Folienproben gestanzt. Derart wurden mindestens 3 Folienproben vorbereitet.

### Füllen

Die wie oben vorbereiteten Löcher wurden mit Hilfe einer Schablone von der Christian Koenen GmbH und einem EKRA Microtronic II-Drucker (Modell M2H) gefüllt. Die Dicke der Schablone betrug 100 µm. Die Öffnungen der Schablone hatten die gleichen Abmessungen und Positionen wie die Löcher, welche wie oben beschrieben in die Grünkörperfolie gestanzt worden waren. Die Druckparameter waren 50 N Rakeldruck, Rakelgeschwindigkeit vorwärts 25 mm/S, Rakelgeschwindigkeit rückwärts 25 mm/s und Abriss (snap off) 0,0 mm. Der Rakelkreis wurde so eingestellt, dass Pastenmaterial sowohl bei der Vorwärtsbewegung als auch bei der Rückwärtsbewegung eingebracht wurde. Dabei wurde eine Füllung von etwa 200 µm Dicke nach dem Drucken (nass) und etwa 150 µm Dicke nach dem Trocken erreicht.

Gemäß dem oben beschrieben Füllschritt wurde eine erste und eine zweite Folienprobe zunächst mit der Metallpaste angefüllt. Hierbei wurde der Füllschritt nur so oft wiederholt, dass das Loch in der Folie nicht vollständig gefüllt war. Zum vollständigen Füllen des Lochs wurden weitere Füllschritte mit der Cermetpaste durchgeführt. Ferner wurden 1 bis 5 weitere Folienproben durch mehrfaches Ausführen des obigen Füllschrittes mit der Cermetpaste vollständig gefüllt.

### Trocknen

10 Minuten nach dem Füllen der Proben wurden diese in einen Trockner HHG-2 (von BTU International Inc.) eingebracht und dort für 10 Minuten bei 80°C getrocknet.

### Laminieren

Die 3 bis 7 Lagen Grünkörperfolie mit wie oben beschrieben gefüllten Löchern wurden mit einem Metallausrichtungswerkzeug gestapelt und in einem Ölbad bei 70°C mit einem Druck von 350 bar für 10 Minuten isostatisch verpresst (Laminator-CE-1 von Autoclave Engineers eine Abteilung von Snap-tite Inc.), um die gewünschte Bauteildicke zu erreichen. Hierbei wurde so gestapelt, dass die erste und zweite Folienprobe jeweils äußere Schichten darstellten und die Metallpastenfüllungen dieser Proben jeweils nach außen wiesen und sich zwischen den Metallpastenfüllungen eine durchgehende Cermetpastenfüllung befand.

### Brennen

Das wie oben erhaltene Laminat von Grünkörperfolien wurde in einem Hochtemperatur-Kammerofen (FHT-175-10-12 der Arnold Schröder Industrieöfen GmbH), geeignet für eine Maximaltemperatur von 1750°C mit einer Kammergröße von 200 mm × 250 mm × 200 mm gebrannt. Dazu wurde das Laminat auf einem Stück poröser Keramik (KERALPOR 99 from Keramische Folien GmbH) in der Ofenkammer platziert. Der Brennvorgang erfolgte unter atmosphärischen Normalbedingungen. Die Temperatur wurde von 25 auf 450°C bei einer Rate von 30°C/h erhöht. Dann wurde die Temperatur bei 450°C für 5 h konstant gehalten, um die organischen Komoponenten in dem Grünkörperlaminat wegzubrennen. Anschließend wurde die Temperatur auf eine Maximaltempoeratur in einem Bereich von 1510 bis 1650°C bei einer RAte von 450°C/h erhöht und bei diesem Wert für eine Haltedauer in einem Bereich von 1 bis 5 Stunden konstant gehalten. Danach wurde die Temperatur bei einer Kühlrate von 450°C/h oder der natürlichen Kühlrate, welche langsamer war, auf Raumtemperatur abgesenkt.

### Nachbehandlung

Nach dem Brennen wurden die Proben abgeschliffen und mit einem Laser auf die gewünschten Abmessungen zugeschnitten.

Es zeigen:
- Figur 1a): einen schematischen Querschnitt durch eine erfindungsgemäße Vorrichtung;
- Figur 1b): einen schematischen Querschnitt durch eine weitere erfindungsgemäße Vorrichtung;
- Figur 2a): einen schematischen Querschnitt durch eine weitere erfindungsgemäße Vorrichtung;
- Figur 2b): einen schematischen Querschnitt durch eine weitere erfindungsgemäße Vorrichtung;
- Figur 3: einen schematischen Querschnitt durch eine weitere erfindungsgemäße Vorrichtung;
- Figur 4: einen schematischen Querschnitt durch eine weitere erfindungsgemäße Vorrichtung;
- Figur 5: einen schematischen Querschnitt durch eine weitere erfindungsgemäße Vorrichtung;
- Figur 6: einen schematischen Querschnitt durch eine weitere erfindungsgemäße Vorrichtung;
- Figur 7: ein Ablaufdiagramm eines Verfahrens;
- Figur 8: einen schematischer Querschnitt durch ein erfindungsgemäßes elektrisches Gerät,
- Figur 9: ein Ablaufdiagramm eines weiteren Verfahrens; und
- Figur 10: einen schematischer Querschnitt durch eine nicht erfindungsgemäße elektrische Durchführung.

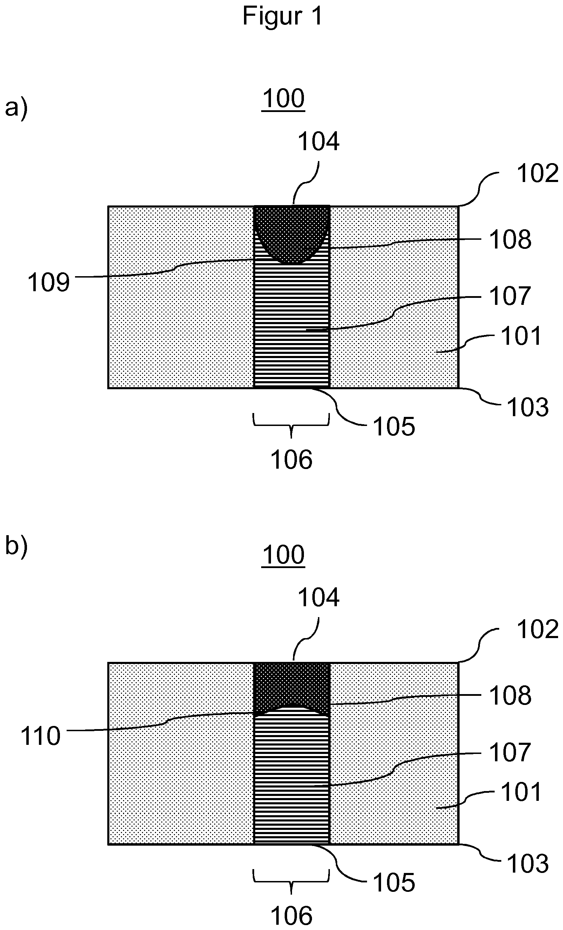

Figur 1a) zeigt einen schematischen Querschnitt durch eine erfindungsgemäße Vorrichtung 100. Die Vorrichtung 100 beinhaltet einen Keramikkörper 101, welcher wiederum eine erste Oberfläche 103 und eine weitere Oberfläche 102 beinhaltet. Der Keramikkörper 101 besteht aus Al₂O₃. Die erste Oberfläche 103 liegt der weiteren Oberfläche 102 gegenüber. Die erste Oberfläche 103 und die weitere Oberfläche 102 sind zwei Stirnflächen des Keramikkörpers 101. Die erste Oberfläche 103 beinhaltet eine erste Öffnung 105, welche kreisrund rund ist. Die weitere Oberfläche 102 beinhaltet eine weitere Öffnung 104, welche ebenfalls kreisrund ist. Die erste Öffnung 105 und die weitere Öffnung 104 werden durch einen zylinderförmigen, sich durch den Keramikkörper 101 erstreckenden Tunnel 106 verbunden. Die erste Öffnung 105 und die weitere Öffnung 104 sind Stirnflächen des Tunnels 106. Die erste Öffnung 105, die weitere Öffnung 104 und der Tunnel 106 sind immaterielle Merkmale. Sie begrenzen einen Hohlraum im Keramikkörper 101. Der Tunnel 106 ist mit einer Tunnelfüllung 106 vollständig gefüllt. Somit ist der Tunnel 106 auch durch die Tunnelfüllung 106 verschlossen. Der Tunnel 106 ist hermetisch dicht durch die Tunnelfüllung 106 verschlossen. Die Tunnelfüllung 106 besteht aus einem ersten Bestandteil 107 und einem zweiten Bestandteil 108. Der erste Bestandteil 107 und der zweite Bestandteil sind miteinander verbunden; sie grenzen direkt aneinander an. Eine der weiteren Öffnung 104 zugewandte Oberfläche der Tunnelfüllung 106 ist eine Oberfläche des zweiten Bestandteils 108. Hier füllt der zweite Bestandteil 108 die weitere Öffnung 104 vollständig aus und verschließt die weitere Öffnung 104 bündig zu der weiteren Oberfläche 102. Der erste Bestandteil 107 und der zweite Bestandteil 108 sind elektrisch leitend. Der erste Bestandteil 107 ist ein Cermet aus 60 Vol.-% Al₂O₃ und 40 Vol.-% Platin, jeweils bezogen auf das Volumen des ersten Bestandteils 107. Der zweite Bestandteil 108 ist ein Cermet aus 20 Vol.-% Al₂O₃ und 80 Vol.-% Platin, jeweils bezogen auf das Volumen des zweiten Bestandteils 108. Es ergibt sich, dass eine erste elektrische Leitfähigkeit des ersten Bestandteils um 1·10⁶ S/m mehr ist als eine weitere elektrische Leitfähigkeit des Keramikkörpers 101. Ferner ist eine zweite elektrische Leitfähigkeit des zweiten Bestandteils 108 um 1·10⁵ S/m mehr als die erste elektrische Leitfähigkeit. Hierbei sind die Tunnelfüllung 106, aus dem ersten Bestandteil 107 und dem zweiten Bestandteil 108, und der Keramikkörper 101 einstückig ausgebildet. Die Tunnelfüllung 106 und der Keramikkörper 101 wurden gemeinsam aus einem Grünkörper gebrannt. Es gibt keine nachträglich erzeugte Intermaterialverbindung zwischen der Tunnelfüllung 106 und dem Keramikkörper 101 sowie zwischen dem ersten Bestandteil 107 und dem zweiten Bestandteil 108. Die vorgenannten Komponenten stellen lediglich räumliche Bereiche unterschiedlicher Materialzusammensetzung der Vorrichtung 100 dar. Der erste Bestandteil 107 bildet eine erste Bestandteilschicht, welche in dem Tunnel 106 eine zweite Bestandteilschicht, gebildet durch den zweiten Bestandteil 108, überlagert. Die beiden Bestandteilschichten grenzen direkt an einer ersten Bestandteiloberfläche 109 des ersten Bestandteils 107 und einer zweiten Bestandteiloberfläche 109 des zweiten Bestandteils 108 aneinander an. Hierbei ist die erste Bestandteiloberfläche 109 konkav und die zweite Bestandteiloberfläche 109 konvex.

Figur 1b) zeigt einen schematischen Querschnitt durch eine weitere erfindungsgemäße Vorrichtung 100. Die Vorrichtung 100 ist identisch zu der Vorrichtung 100 aus Figur 1a), abgesehen davon, dass in Figur 1b) die erste Bestandteiloberfläche 110 konvex ist und die zweite Bestandteiloberfläche 110 konkav ist.

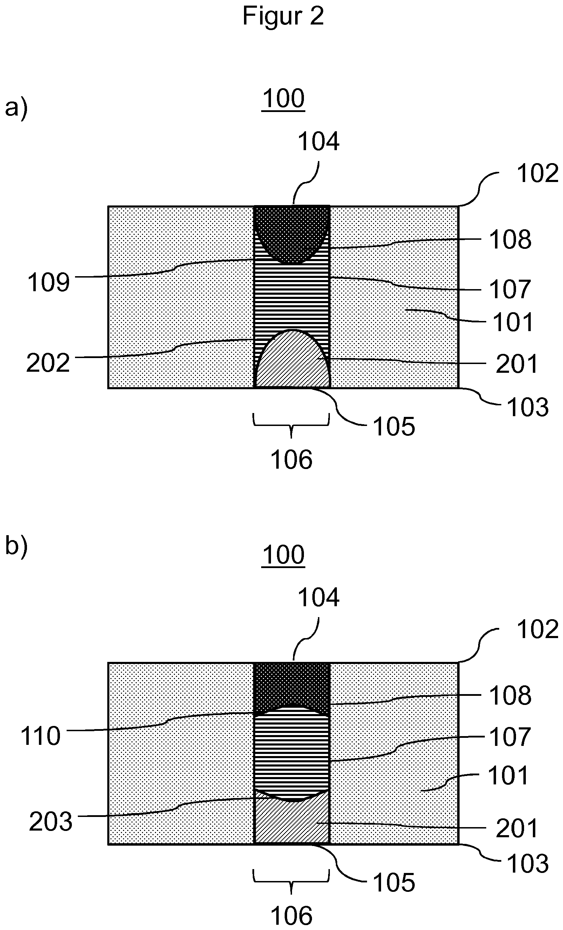

Figur 2a) zeigt einen schematischen Querschnitt durch eine weitere erfindungsgemäße Vorrichtung 100. Die Vorrichtung 100 ist identisch zu der Vorrichtung 100 aus Figur 1a), abgesehen davon, dass in Figur 2a) die Tunnelfüllung 106 weiter einen dritten Bestandteil 201 beinhaltet. Eine Materialzusammensetzung des dritten Bestandteils 201 ist identisch zu der des zweiten Bestandteils 108. Demnach ist auch der dritte Bestandteil 201 elektrisch leitend. Der dritte Bestandteil 201 und der erste Bestandteil 107 sind miteinander verbunden; sie grenzen direkt aneinander an. Eine der ersten Öffnung 105 zugewandte Oberfläche der Tunnelfüllung 106 ist eine Oberfläche des dritten Bestandteils 201. Hier füllt der dritte Bestandteil 201 die erste Öffnung 105 vollständig aus und verschließt die erste Öffnung 105 bündig zu der ersten Oberfläche 103. Eine dritte elektrische Leitfähigkeit des dritten Bestandteils 201 ist gleich der zweiten elektrischen Leitfähigkeit. Die erste Bestandteilschicht überlagert in dem Tunnel 106 eine dritte Bestandteilschicht, gebildet durch den dritten Bestandteil 201. Die erste Bestandteilschicht und die dritte Bestandteilschicht grenzen direkt an einer weiteren ersten Bestandteiloberfläche 202 des ersten Bestandteils 107 und einer dritten Bestandteiloberfläche 202 des dritten Bestandteils 201 aneinander an. Hierbei ist die weitere erste Bestandteiloberfläche 202 konkav und die dritte Bestandteiloberfläche 202 konvex.

Figur 2b) zeigt einen schematischen Querschnitt durch eine weitere erfindungsgemäße Vorrichtung 100. Die Vorrichtung 100 ist identisch zu der Vorrichtung 100 aus Figur 2a), abgesehen davon, dass in Figur 2b) die erste Bestandteiloberfläche 110 konvex ist und die zweite Bestandteiloberfläche 110 konkav ist, sowie die weitere erste Bestandteiloberfläche 203 konvex ist und die dritte Bestandteiloberfläche 203 konkav ist.

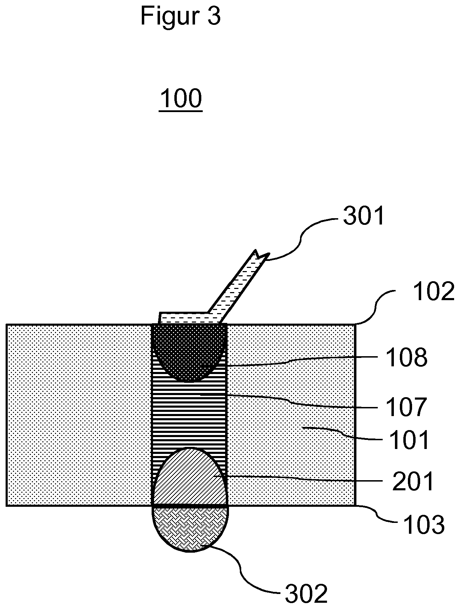

Figur 3 zeigt einen schematischen Querschnitt durch eine weitere erfindungsgemäße Vorrichtung 100. Die Vorrichtung 100 ist identisch zu der Vorrichtung 100 aus Figur 2a), abgesehen davon, dass in Figur 3 der zweite Bestandteil 108 und der dritte Bestandteil 201 jeweils mit einem elektrischen Leiter 301, 302 verbunden sind. An den zweiten Bestandteil 108 ist ein Bonddraht 301 gebondet. An den dritten Bestandteil 302 ist ein Lot 302 gelötet. Über dieses Lot kann ein Draht oder Pin angelötet werden. Somit kann das Durchführungselement der Vorrichtung 100, also hier die Tunnelfüllung 106, direkt ohne weitere Komponente elektrisch leitend verbunden werden.

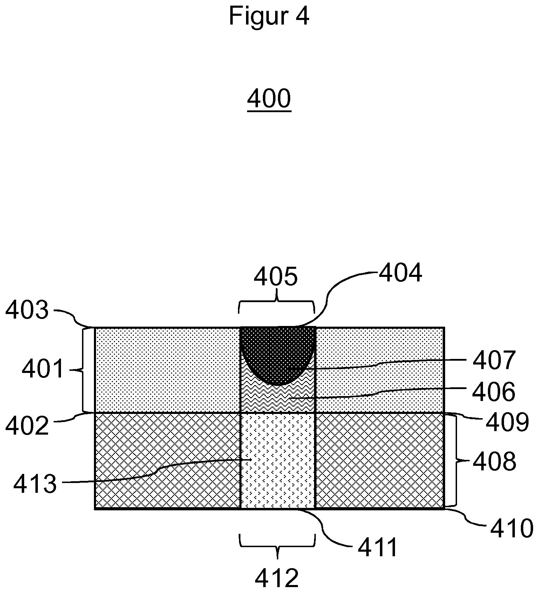

Figur 4 zeigt einen schematischen Querschnitt durch eine weitere erfindungsgemäße Vorrichtung 400. Die Vorrichtung 400 ist ein Grünkörper. Die Vorrichtung 400 beinhaltet eine erste Keramikvorläuferschicht 401 und eine zweite Keramikvorläuferschicht 408. Die erste Keramikvorläuferschicht 401 beinhaltet eine erste Schichtoberfläche 402 und eine weitere Schichtoberfläche 403, wobei die erste Schichtoberfläche 402 der weiteren Schichtoberfläche 403 gegenüber liegt. Ferner beinhaltet die erste Schichtoberfläche 402 eine erste Öffnung und die weitere Schichtoberfläche 403 eine weitere Öffnung 404. Die erste Öffnung und die weitere Öffnung 404 sind durch einen zylinderförmigen, sich durch die erste Keramikvorläuferschicht 401 erstreckenden ersten Tunnel 405 verbunden. Der erste Tunnel 405 beinhaltet eine erste Tunnelfüllung 405 vollständig und ist vollständig von der ersten Tunnelfüllung 405 ausgefüllt und somit verschlossen. Die erste Tunnelfüllung 405 besteht aus einer ersten Zusammensetzung 406 und einer zweiten Zusammensetzung 407. Die erste Zusammensetzung 406 ist eine Cermetpaste, bestehend aus 18 Gew.-% eines Al₂O₃-Pulver, 73 Gew.-% eines Platinpulvers und 9 Gew.-% eines organischen Vehikels, jeweils bezogen auf das Gewicht der ersten Zusammensetzung 406. Die zweite Zusammensetzung 407 ist eine Cermetpaste, bestehend aus1,8 Gew.-% eines Al₂O₃-Pulver, 89,2 Gew.-% eines Platinpulvers und 9 Gew.-% eines organischen Vehikels, jeweils bezogen auf das Gewicht der zweiten Zusammensetzung 407. Die weitere Öffnung 404 ist bündig zu der weiteren Schichtoberfläche 403 und vollständig mit der zweiten Zusammensetzung 407 ausgefüllt und verschlossen. Die zweite Keramikvorläuferschicht 408 beinhaltet eine erste Schichtoberfläche 410 und eine weitere Schichtoberfläche 409, wobei die erste Schichtoberfläche 410 der weiteren Schichtoberfläche (409) gegenüber liegt. Die erste Schichtoberfläche 410 beinhaltet eine erste Öffnung 411 und die weitere Schichtoberfläche 409 beinhaltet eine weitere Öffnung. Die erste Öffnung 411 und die weitere Öffnung sind durch einen zylinderförmigen, sich durch die zweite Keramikvorläuferschicht 408 erstreckenden zweiten Tunnel 412 verbunden. Der zweite Tunnel 412 ist vollständig mit einer zweiten Tunnelfüllung 412 ausgefüllt und verschlossen. Die zweite Tunnelfüllung 412 besteht aus einer zweiten ersten Zusammensetzung 413. Die zweite erste Zusammensetzung 412 ist eine Cermetpaste, deren Inhalt identisch zu dem der ersten Zusammensetzung 406 ist. Die weitere Schichtoberfläche 409 der zweiten Keramikvorläuferschicht 408 ist mit der ersten Schichtoberfläche 402 der ersten Keramikvorläuferschicht 401 so kontaktiert ist, dass die erste Tunnelfüllung 405 und die zweite Tunnelfüllung 412 kontaktiert sind. Hier liegen die erste Öffnung der ersten Keramikvorläuferschicht 401 und die weitere Öffnung der zweiten Keramikvorläuferschicht 408 deckungsgleich übereinander. Die erste Keramikvorläuferschicht 401 und die zweite Keramikvorläuferschicht 408 sind aufeinander laminiert und bilden zusammen mit ihren jeweiligen Tunnelfüllungen 405, 412 einen Grünkörper. Die erste Keramikvorläuferschicht 401 und die zweite Keramikvorläuferschicht 408 haben jeweils eine Schichtdicke von 400 µm. Ferner grenzen die erste Zusammensetzung 407 und die zweite Zusammensetzung 406 entlang einer konvexen Oberfläche der zweiten Zusammensetzung 407 und einer dazu komplementären konkaven Oberfläche der ersten Zusammensetzung 406 aneinander an.

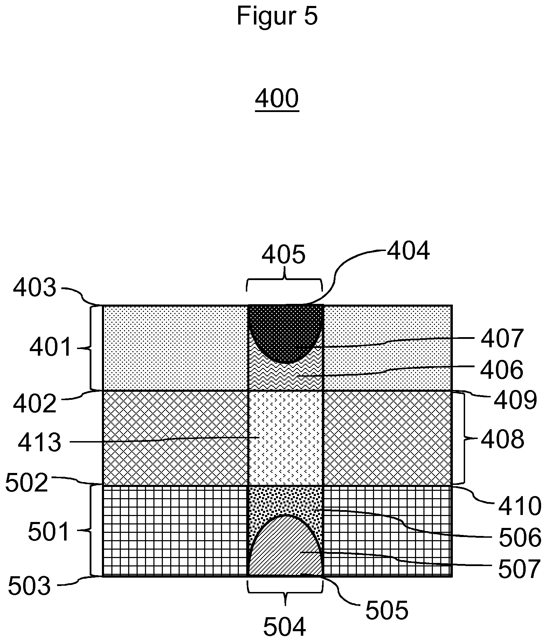

Figur 5 zeigt einen schematischen Querschnitt durch eine weitere erfindungsgemäße Vorrichtung 400. Die Vorrichtung 400 ist identisch zu der Vorrichtung 400 aus Figur 4, wobei die Vorrichtung 400 der Figur 5 weiter eine dritte Keramikvorläuferschicht 501 beinhaltet. Die dritte Keramikvorläuferschicht 501 beinhaltet eine erste Schichtoberfläche 503 und eine weitere Schichtoberfläche 502, wobei die erste Schichtoberfläche 503 der weiteren Schichtoberfläche 502 gegenüber liegt. Die erste Schichtoberfläche 503 beinhaltet eine erste Öffnung 505 und die weitere Schichtoberfläche 502 eine weitere Öffnung. Die erste Öffnung 505 und die weitere Öffnung sind durch einen zylinderförmigen, sich durch die dritte Keramikvorläuferschicht 501 erstreckenden dritten Tunnel 504 verbunden. Der dritte Tunnel 504 beinhaltet eine dritte Tunnelfüllung 504 vollständig und ist durch die dritte Tunnelfüllung (504) vollständig ausgefüllt und verschlossen. Die dritte Tunnelfüllung 504 besteht aus einer dritten ersten Zusammensetzung 506 und einer weiteren zweiten Zusammensetzung 507. Die weitere zweite Zusammensetzung 507 füllt die erste Öffnung 505 vollständig aus und verschließt diese bündig zu der ersten Schichtoberfläche 503. Die dritte erste Zusammensetzung 506 ist eine Cermetpaste, deren Inhalt identisch zu dem der ersten Zusammensetzung 406 ist. Die weitere zweite Zusammensetzung 507 ist eine Cermetpaste, deren Inhalt identisch zu dem der zweiten Zusammensetzung 407 ist. Die erste Schichtoberfläche 410 der zweiten Keramikvorläuferschicht 408 ist durch die weitere Schichtoberfläche 502 der dritten Keramikvorläuferschicht 501 überlagert. Die dritte Keramikvorläuferschicht 501 hat ebenfalls eine Schichtdicke von 400 µm und ist auf die zweite Keramikvorläuferschicht 408 laminiert. Ferner grenzen die dritte erste Zusammensetzung 506 und die weitere zweite Zusammensetzung 507 entlang einer konvexen Oberfläche der weiteren zweiten Zusammensetzung 507 und einer dazu komplementären konkaven Oberfläche der dritten ersten Zusammensetzung 506 aneinander an.

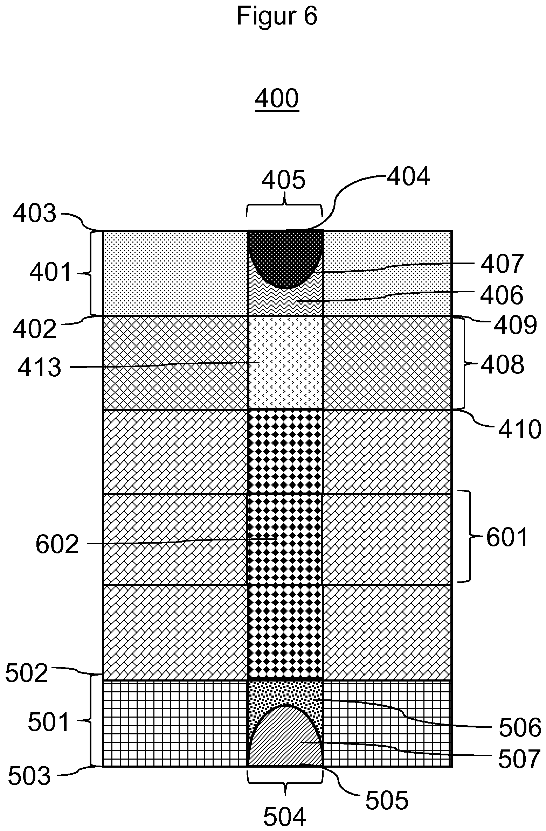

Figur 6 zeigt einen schematischen Querschnitt durch eine weitere erfindungsgemäße Vorrichtung 400. Die Vorrichtung 400 ist identisch zu der Vorrichtung 400 aus Figur 5, wobei die Vorrichtung 400 der Figur 6 weiter drei weitere Keramikvorläuferschichten 601 beinhaltet. Die weiteren Keramikvorläuferschichten 601 befinden sich zwischen der zweiten Keramikvorläuferschicht 408 und der dritten Keramikvorläuferschicht 501. Alle Keramikvorläuferschichten 401, 408, 501, 601 der Vorrichtung 400 sind aufeinander laminiert. Die weiteren Keramikvorläuferschichten sind jeweils 400 µm dick und beinhalten jeweils einen weiteren Tunnel, welcher mit einer Cermetpaste, die identisch zu der Cermetpaste, welche die erste Zusammensetzung 406 ist, ist. In der Vorrichtung 400 liegen alle Tunnel deckungsgleich übereinander, so dass sich von der weiteren Schichtoberfläche 403 der ersten Keramikvorläuferschicht 401 zu der ersten Schichtoberfläche 503 der dritten Keramikvorläuferschicht 501 ein Pfad aus der zweiten Zusammensetzung 407, der ersten Zusammensetzung 406, der zweiten ersten Zusammensetzung 413, den weiteren ersten Zusammensetzungen 602, der dritten ersten Zusammensetzung 506 und der weiteren zweiten Zusammensetzung 507 ergibt.

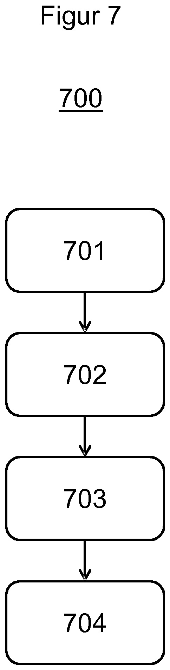

Figur 7 zeigt ein Ablaufdiagramm eines Verfahrens 700. Das Verfahren 700 beinhaltet als Verfahrensschritt a) 701 ein Bereitstellen einer ersten Keramikvorläuferschicht, beinhaltend eine erste Schichtoberfläche und eine weitere Schichtoberfläche, wobei die erste Schichtoberfläche der weiteren Schichtoberfläche gegenüber liegt. Die erste Schichtoberfläche beinhaltet eine erste Öffnung und die weitere Schichtoberfläche eine weitere Öffnung. Die erste Öffnung und die weitere Öffnung sind durch einen ersten Tunnel verbunden. Der erste Tunnel beinhaltet eine erste Tunnelfüllung mindestens teilweise und ist durch die erste Tunnelfüllung verschlossen. Die erste Tunnelfüllung beinhaltet eine erste Zusammensetzung und eine zweite Zusammensetzung. Eine bündig zu der weiteren Schichtoberfläche in der weiteren Öffnung liegende Oberfläche der ersten Tunnelfüllung ist eine Oberfläche der zweiten Zusammensetzung. Die erste Zusammensetzung hat einen ersten Zusammensetzungsmetallanteil bezogen auf das Gewicht der ersten Zusammensetzung und die zweite Zusammensetzung hat einen zweiten Zusammensetzungsmetallanteil bezogen auf das Gewicht der zweiten Zusammensetzung. Der zweite Zusammensetzungsmetallanteil ist um mindestens 10 Gew.-% mehr als der erste Zusammensetzungsmetallanteil. Die erste Keramikvorläuferschicht des Verfahrens 700 ist identisch zu der ersten Keramikvorläuferschicht 401 der Figur 4. In einem Verfahrensschritt b) 702 wird eine zweite Keramikvorläuferschicht, beinhaltend eine erste Schichtoberfläche und eine weitere Schichtoberfläche, bereitgestellt. Die erste Schichtoberfläche liegt der weiteren Schichtoberfläche gegenüber. Die erste Schichtoberfläche beinhaltet eine erste Öffnung und die weitere Schichtoberfläche beinhaltet eine weitere Öffnung. Die erste Öffnung und die weitere Öffnung sind durch einen zweiten Tunnel verbunden. Der zweite Tunnel beinhaltet eine zweite Tunnelfüllung und ist durch die zweite Tunnelfüllung verschlossen. Die zweite Tunnelfüllung beinhaltet eine zweite erste Zusammensetzung. Die zweite erste Zusammensetzung hat einen zweiten ersten Zusammensetzungsmetallanteil bezogen auf das Gewicht der zweiten ersten Zusammensetzung. Der zweite Zusammensetzungsmetallanteil ist um mindestens 10 Gew.-% mehr als der zweite erste Zusammensetzungsmetallanteil. Die zweite Keramikvorläuferschicht des Verfahrens 700 ist identisch zu der zweiten Keramikvorläuferschicht 408 der Figur 4. In einem Verfahrensschritt c) werden die weitere Schichtoberfläche der zweiten Keramikvorläuferschicht und die erste Schichtoberfläche der ersten Keramikvorläuferschicht so aufeinander laminiert, dass die erste Tunnelfüllung und die zweite Tunnelfüllung dabei kontaktiert werden. Das Laminieren erfolgt in einem Ölbad mit der Temperatur von 70°C als isostatisches Pressen. In einem Verfahrensschritt d) 704 werden die erste Keramikvorläuferschicht und die zweite Keramikvorläuferschicht gemeinsam gebrannt. Hierbei beinhaltet das Brennen eine Maximaltemperatur von 1650°C, welche für eine Haltedauer von 2,5 h gehalten wird.

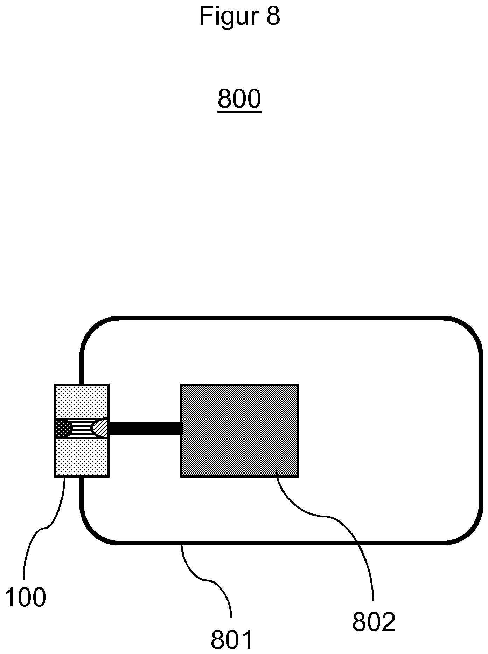

Figur 8 zeigt einen schematischen Querschnitt durch ein erfindungsgemäßes elektrisches Gerät 800. Das elektrische Gerät 800 ist ein implantierbarer Herzschrittmacher. Das elektrische Gerät 800 beinhaltet ein Gehäuse 801 aus Titan, welches eine Gehäuseöffnung beinhaltet. In die Gehäuseöffnung ist über einen nicht dargestellten Titanflansch die Vorrichtung 100 gemäß Figur 2a) eingeschweißt. Somit ist das Gehäuse 801 hermetisch dicht verschlossen. Das Gehäuse 801 beinhaltet in seinem Inneren einen elektrischen Impulsgeber 802, welcher über einen Bonddraht 301 durch Bonden mit dem dritten Bestandteil 201 der Vorrichtung 100 elektrisch leitend verbunden ist. Die Vorrichtung 100 ist eine elektrische Cermetdurchführung in dem Herzschrittmacher.

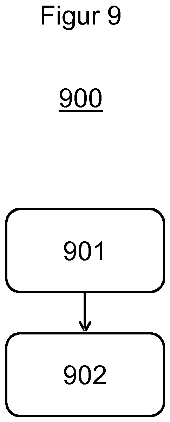

Figur 9 zeigt ein Ablaufdiagramm eines weiteren Verfahrens 900. Das Verfahren 900 beinhaltend einen Verfahrensschritt a) Bereitstellen des Herzschrittmachers 800 aus Figur 8 und einen Verfahrensschritt b) 902 Implantieren des Herzschrittmachers 800 in einen menschlichen Patienten.

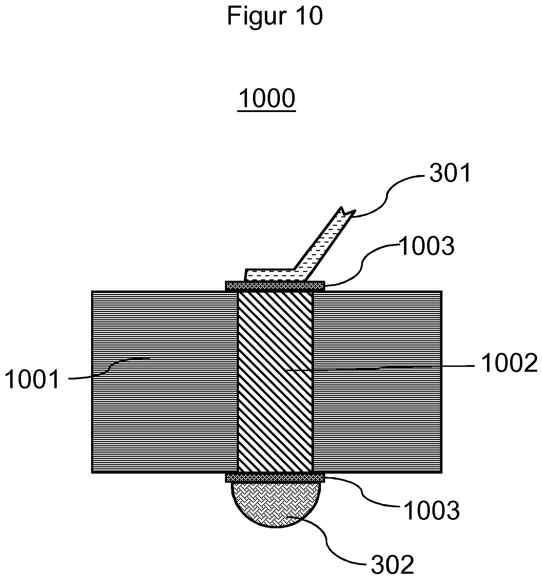

Figur 10 zeigt einen schematischen Querschnitt durch eine nicht erfindungsgemäße elektrische Durchführung 1000. Die Durchführung 1000 beinhaltet einen Keramikring 1001 als Isolationskörper und ein Cermet 1002 als elektrisch leitendes Durchführungselement. Ein Metallgehalt des Cermets 1002 ist so gewählt, dass eine optimale Verbindung zu dem Keramikring 1001 erzielt wird. Um das Cermet mit einem Bonddraht 301 und einem Lot 302 elektrisch verbinden zu können, wurden metallhaltige Oberflächen 1003 mit einem gegenüber dem Metallgehalt des Cermets 1002 erhöhten Metallgehalt auf das Cermet 1002 aufgebracht. Die Kontaktierung des Durchführungselements erfolgt demnach über die zusätzlichen metallhaltigen Oberflächen 1003.

### LISTE DER BEZUGSZEICHEN

- **100**: erfindungsgemäße Vorrichtung
- **101**: Keramikkörper
- **102**: weitere Oberfläche
- **103**: erste Oberfläche
- **104**: weitere Öffnung
- **105**: erste Öffnung
- **106**: Tunnel / Tunnelfüllung
- **107**: erster Bestandteil
- **108**: zweiter Bestandteil
- **109**: konkave erste Bestandteiloberfläche / konvexe zweite Bestandteiloberfläche
- **110**: konvexe erste Bestandteiloberfläche / konkave zweite Bestandteiloberfläche
- **201**: dritter Bestandteil
- **202**: konkave weiter erste Bestandteiloberfläche / konvexe dritte Bestandteiloberfläche
- **203**: konvexe weitere erste Bestandteiloberfläche / konkave dritte Bestandteiloberfläche
- **301**: Bonddraht
- **302**: Lot
- **400**: weitere erfindungsgemäße Vorrichtung
- **401**: erste Keramikvorläuferschicht
- **402**: erste Schichtoberfläche der ersten Keramikvorläuferschicht
- **403**: weitere Schichtoberfläche der ersten Keramikvorläuferschicht
- **404**: weitere Öffnung der ersten Keramikvorläuferschicht
- **405**: erster Tunnel / erste Tunnelfüllung
- **406**: erste Zusammensetzung
- **407**: zweite Zusammensetzung
- **408**: zweite Keramikvorläuferschicht
- **409**: weitere Schichtoberfläche der zweiten Keramikvorläuferschicht
- **410**: erste Schichtoberfläche der zweiten Keramikvorläuferschicht
- **411**: erste Öffnung der zweiten Keramikvorläuferschicht
- **412**: zweiter Tunnel / zweite Tunnelfüllung
- **413**: zweite erste Zusammensetzung
- **501**: dritte Keramikvorläuferschicht
- **502**: weitere Schichtoberfläche der dritten Keramikvorläuferschicht
- **503**: erste Schichtoberfläche der dritten Keramikvorläuferschicht
- **504**: dritter Tunnel / dritte Tunnelfüllung
- **505**: erste Öffnung der dritten Keramikvorläuferschicht
- **506**: dritte erste Zusammensetzung
- **507**: weitere zweite Zusammensetzung
- **601**: weitere Keramikvorläuferschicht
- **602**: weitere erste Zusammensetzung
- **700**: erfindungsgemäßes Verfahren
- **701**: Verfahrensschritt a) des erfindungsgemäßen Verfahrens
- **702**: Verfahrensschritt b) des erfindungsgemäßen Verfahrens
- **703**: Verfahrensschritt c) des erfindungsgemäßen Verfahrens
- **704**: Verfahrensschritt d) des erfindungsgemäßen Verfahrens
- **800**: erfindungsgemäßes elektrisches Gerät
- **801**: Gehäuse
- **802**: elektrischer Impulsgeber
- **900**: weiteres erfindungsgemäßes Verfahren
- **901**: Verfahrensschritt a) des weiteren erfindungsgemäßen Verfahrens
- **902**: Verfahrensschritt b) des weiteren erfindungsgemäßen Verfahrens
- **1000**: nicht erfindungsgemäße elektrische Durchführung
- **1001**: Keramikring
- **1002**: Cermet
- **1003**: metallhaltige Oberfläche

## Patentansprüche

1. Eine Vorrichtung (100) beinhaltend einen Keramikkörper (101), beinhaltend eine erste Oberfläche (103) und eine weitere Oberfläche (102);
wobei die erste Oberfläche (103) der weiteren Oberfläche (102) gegenüber liegt;
wobei die erste Oberfläche (103) eine erste Öffnung (105) beinhaltet;
wobei die weitere Oberfläche (102) eine weitere Öffnung (104) beinhaltet;
wobei die erste Öffnung (105) und die weitere Öffnung (104) durch einen Tunnel (106) verbunden sind;
wobei der Tunnel (106) eine Tunnelfüllung (106) mindestens teilweise beinhaltet und durch die Tunnelfüllung (106) verschlossen ist;
wobei die Tunnelfüllung (106) einen ersten Bestandteil (107) und einen zweiten Bestandteil (108) beinhaltet;
wobei der erste Bestandteil (107) ein Cermet beinhaltet;
wobei der erste Bestandteil (107) und der zweite Bestandteil (108) elektrisch leitend miteinander verbunden sind;
wobei eine der weiteren Öffnung (104) zugewandte Oberfläche der Tunnelfüllung (106) mindestens teilweise eine Oberfläche des zweiten Bestandteils (108) ist;
wobei der erste Bestandteil (107) **gekennzeichnet ist durch** eine erste elektrische Leitfähigkeit;
wobei der zweite Bestandteil (108) **gekennzeichnet ist durch** eine zweite elektrische Leitfähigkeit;
wobei der Keramikkörper (101) **gekennzeichnet ist durch** eine weitere elektrische Leitfähigkeit;
wobei die erste elektrische Leitfähigkeit um mindestens 1·10⁵ S/m mehr ist als die weitere elektrische Leitfähigkeit;
wobei die zweite elektrische Leitfähigkeit sich von der ersten elektrischen Leitfähigkeit um mindestens 5·10⁴ S/m unterscheidet.

2. Die Vorrichtung (100) nach Anspruch 1, wobei die zweite elektrische Leitfähigkeit mehr ist als die erste elektrische Leitfähigkeit.

3. Die Vorrichtung (100) nach Anspruch 1 oder 2, wobei der erste Bestandteil (107) einen ersten Metallanteil bezogen auf das Gewicht des ersten Bestandteils (107) hat;
wobei der zweite Bestandteil (108) einen zweiten Metallanteil bezogen auf das Gewicht des zweiten Bestandteils (108) hat;
wobei der zweite Metallanteil um mindestens 5 Gew.-% mehr ist als der erste Metallanteil.

4. Die Vorrichtung (100) nach einem der vorhergehenden Ansprüche, wobei die Tunnelfüllung (106) einen dritten Bestandteil (201) beinhaltet;
wobei der dritte Bestandteil (201) elektrisch leitend mit dem ersten Bestandteil (107) oder dem zweiten Bestandteil (108) oder beiden verbunden ist;
wobei eine der ersten Öffnung (105) zugewandte Oberfläche der Tunnelfüllung (106) mindestens teilweise eine Oberfläche des dritten Bestandteils (201) ist;
wobei der dritte Bestandteil (201) **gekennzeichnet ist durch** eine dritte elektrische Leitfähigkeit;
wobei die dritte elektrische Leitfähigkeit sich von der ersten elektrischen Leitfähigkeit um mindestens 5·10⁴ S/m unterscheidet.

5. Die Vorrichtung (100) nach Anspruch 4, wobei eines ausgewählt aus der Gruppe bestehend aus dem ersten Bestandteil (107), dem zweiten Bestandteil (108) und dem dritten Bestandteil (201), oder eine Kombination aus mindestens zwei davon ein Cermet ist.

6. Ein elektrisches Gerät (800), beinhaltend eine Vorrichtung (100) nach einem der Ansprüche 1 bis 5 und ein Gehäuse (801), beinhaltend eine Gehäuseöffnung;
wobei die Gehäuseöffnung die Vorrichtung (100) umrahmt.

7. Das elektrische Gerät (800) nach Anspruch 6, wobei das elektrische Gerät (800) ein implantierbares elektrisches medizinisches Gerät (800) ist.

8. Eine Verwendung der Vorrichtung (100) nach einem der Ansprüche 1 bis 5 zu einem elektrischen Verbinden eines Inneren eines Gehäuses (801) mit einem Äußeren des Gehäuses (801).

## Claims

1. An apparatus (100) comprising a ceramic body (101) including a first surface (103) and a further surface (102);
the first surface (103) facing the further surface (102);
the first surface (103) including a first opening (105);
the further surface (102) including a further opening (104);
wherein the first opening (105) and the further opening (104) are connected by a tunnel (106);
wherein the tunnel (106) at least partially includes a tunnel filling (106) and is closed by the tunnel filling (106);
the tunnel filling (106) including a first component (107) and a second component (108);
wherein the first component (107) comprises a cermet;
wherein the first component (107) and the second component (108) are electrically conductively connected to each other;
wherein a surface of the tunnel filling (106) facing the further opening (104) is at least partially a surface of the second component (108);
the first component (107) being **characterized by** a first electrical conductivity;
the second component (108) being **characterized by** a second electrical conductivity;
said ceramic body (101) being **characterized by** further electrical conductivity;
wherein the first electrical conductivity is at least 1·10⁵ S/m more than the further electrical conductivity;
wherein the second electrical conductivity differs from the first electrical conductivity by at least 5·10⁴ S/m.

2. The device (100) according to claim 1, wherein the second electrical conductivity is more than the first electrical conductivity.

3. The apparatus (100) according to claim 1 or 2, wherein the first component (107) has a first metal content based on the weight of the first component (107);
wherein the second component (108) has a second metal content based on the weight of the second component (108);
wherein the second metal portion is at least 5% by weight more than the first metal portion.

4. The apparatus (100) according to one of the preceding claims, wherein the tunnel filling (106) includes a third component (201);
the third component (201) being electrically conductively connected to the first component (107) or the second component (108) or both;
wherein a surface of the tunnel filling (106) facing the first opening (105) is at least partially a surface of the third component (201);
the third component (201) being **characterized by** a third electrical conductivity;
wherein the third electrical conductivity differs from the first electrical conductivity by at least 5·10⁴ S/m.

5. The device (100) according to one of the preceding claims, wherein one selected from the group consisting of the first component (107), the second component (108) and the third component (201), or a combination of at least two thereof, is a cermet.

6. An electrical device (800) including a housing (801) including a housing opening;
the housing opening framing the apparatus (100) according to any one of claims 1 to 5.

7. The electrical device (800) according to Claim 6, where the electrical device (800) is an implantable electrical medical device (800).

8. A use of the apparatus (100) according to any one of claims 1 to 5 for electrically connecting an interior of a housing (801) to an exterior of the housing (801).

## Revendications

1. Appareil (100) comprenant un corps céramique (101) comprenant une première surface (103) et une autre surface (102) ;
la première surface (103) étant vis-à-vis de la surface supplémentaire (102) ;
la première surface (103) comportant une première ouverture (105) ;
la surface supplémentaire (102) comprenant une autre ouverture (104) ;
dans laquelle la première ouverture (105) et l'autre ouverture (104) sont reliées par un tunnel (106) ;
dans lequel le tunnel (106) comprend au moins partiellement un remplissage de tunnel (106) et est fermé par le remplissage de tunnel (106) ;
le remplissage de tunnel (106) comprenant un premier composant (107) et un second composant (108) ;
dans laquelle le premier composant (107) comprend un cermet ;
dans laquelle le premier composant (107) et le second composant (108) sont reliés électriquement l'un à l'autre ;
dans laquelle une surface du remplissage de tunnel (106) tournée vers l'ouverture supplémentaire (104) est au moins partiellement une surface du second composant (108) ; le premier composant (107) étant **caractérisé par** une première conductivité électrique ; le second composant (108) étant **caractérisé par** une seconde conductivité électrique ; ledit corps céramique (101) étant **caractérisé par** une conductivité électrique supplémentaire ;
dans laquelle la première conductivité électrique est supérieure d'au moins 1·10⁵ S/m à la conductivité électrique supplémentaire ;
dans laquelle la seconde conductivité électrique diffère de la première conductivité électrique d'au moins 5·10⁴ S/m.

2. Dispositif (100) selon la revendication 1, dans lequel la deuxième conductivité électrique est supérieure à la première conductivité électrique.

3. Appareil (100) selon la revendication 1 ou 2, dans lequel le premier composant (107) a une première teneur en métal par rapport au poids du premier composant (107) ;
le second composant (108) ayant une deuxième teneur en métal par rapport au poids du second composant (108) ;
dans laquelle la deuxième partie métallique est au moins 5 % en poids de plus que la première partie métallique.

4. Dispositif (100) selon l'une des revendications précédentes, **caractérisé en ce que** le remplissage du tunnel (106) comprend un troisième composant (201) ;
le troisième composant (201) étant connecté électriquement de manière conductrice au premier composant (107) ou au deuxième composant (108) ou aux deux ;
dans laquelle une surface du remplissage de tunnel (106) tournée vers la première ouverture (105) est au moins partiellement une surface du troisième composant (201) ;
le troisième composant (201) étant **caractérisé par** une troisième conductivité électrique;
dans laquelle la troisième conductivité électrique diffère de la première conductivité électrique d'au moins 5·10⁴ S/m.

5. Dispositif (100) selon l'une des revendications précédentes, dans lequel l'un choisi dans le groupe constitué par le premier composant (107), le deuxième composant (108) et le troisième composant (201), ou une combinaison d'au moins deux de ceux-ci, est un cermets.

6. Dispositif électrique (800) comprenant un boîtier (801) comprenant une ouverture de boîtier ;
l'ouverture du boîtier encadrant l'appareil (100) selon l'une quelconque des revendications 1 à 5.

7. Dispositif électrique (800) selon la revendication 6, le dispositif électrique (800) étant un dispositif médical électrique implantable (800).

8. Utilisation de l'appareil (100) selon l'une quelconque des revendications 1 à 5 pour connecter électriquement un intérieur d'un boîtier (801) à un extérieur du boîtier (801).
